(19) Europäisches Patentamt
European Patent Office
Office européen des brevets

(11) Veröffentlichungsnummer: **0 036 388**
**B1**

(12) # EUROPÄISCHE PATENTSCHRIFT

(45) Veröffentlichungstag der Patentschrift: **15.02.84**

(51) Int. Cl.³: **C 09 B 57/04,** C 09 B 26/02, C 07 D 403/12, C 07 D 209/44

(21) Anmeldenummer: **81810082.8**

(22) Anmeldetag: **09.03.81**

(54) **Isoindolinverbindungen, Verfahren zu deren Herstellung und Verwendung.**

(30) Priorität: **13.03.80 CH 1976/80**

(43) Veröffentlichungstag der Anmeldung: **23.09.81 Patentblatt 81/38**

(45) Bekanntmachung des Hinweises auf die Patenterteilung: **15.02.84 Patentblatt 84/7**

(84) Benannte Vertragsstaaten: **CH DE FR GB IT LI**

(56) Entgegenhaltungen: **GB - A - 1 467 595**

(73) Patentinhaber: **CIBA-GEIGY AG, Patentabteilung Postfach, CH-4002 Basel (CH)**

(72) Erfinder: **Iqbal, Abul, Dr., Im Schaiengarten 1, CH-4107 Ettingen (CH)**
Erfinder: **Lienhard, Paul, Dr., Kirschgartenstrasse 14, CH-4402 Frenkendorf (CH)**

EP 0 036 388 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Isoindolinverbindungen, Verfahren zu deren Herstellung und Verwendung

Die Erfindung betrifft ein Verfahren zur Herstellung von 1:1-Metallkomplexen von Isoindolin-azinen der Formel (1)

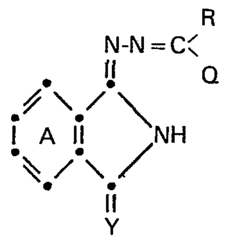 (1)

worin das Metall Zn, Cd, Mn, Co, Fe, Cu oder Ni oder ein Gemisch dieser Metalle ist, der Ring A noch weiter substituiert sein kann,

Y den Rest einer methylenaktiven Verbindung oder eines isocyclischen oder heterocyclischen aromatischen Amins,

R ein Wasserstoffatom, eine Alkyl- oder Arylgruppe und

Q eine Gruppe der Formeln (2), (3) oder (4)

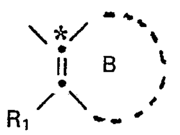 , 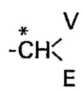 oder -NH-T

(3) (4)

bedeuten, worin

B einen isocyclischen oder heterocyclischen Rest,

$R_1$ eine Hydroxy- oder Mercaptogruppe,

E einen 5- bis 6gliedrigen, ein zum C*-Atom β-ständiges N-Atom enthaltenden und gegebenenfalls annellierten Heteroring, eine Acyl- oder eine gegebenenfalls substituierte Carbamoyl- oder Thiocarbamoylgruppe,

V eine Acyl-, Cyan-, Nitro- oder gegebenenfalls substituierte Carbamoylgruppe und

T einen 5- bis 6gliedrigen, ein zur NH-Gruppe β-ständiges N-Atom enthaltenden und gegebenenfalls annellierten Heteroring oder einen Rest der Formel (5)

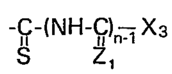 (5)

bedeuten, worin

$Z_1$ für ein O- oder S-Atom, n für die Zahl 1 oder 2 und

$X_3$ für einen Alkyl-, Cycloalkyl-, Aralkyl-, Aryl- oder heterocyclischen Rest oder eine gegebenenfalls durch einen Alkyl-, Cycloalkyl-, Aralkyl-, Aryl- oder Heteroarylrest substituierte Aminogruppe stehen, indem man bei erhöhter Temperatur und in einem polaren organischen Lösungsmittel

a) ein Hydrazon der Formel (6)

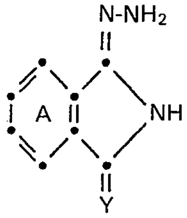 (6)

mit einem Orthocarbonsäureester der Formel (7)

$$C\text{-}R(OR_2)_3 \qquad (7)$$

oder einem Amidin der Formel (8)

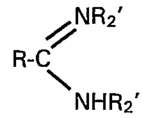 (8)

oder einem Salz eines Amidins der Formel (8) mit einer organischen oder anorganischen Säure zu einem Azin der Formel (9)

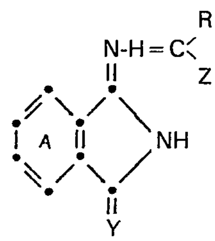 (9)

kondensiert und das erhaltene Kondensationsprodukt in Gegenwart eines Zink, Cadmium, Mangan, Kobalt, Eisen, Kupfer oder Nickel oder ein Gemisch dieser Metalle abgebenden Mittels mit einer Verbindung der Formel (10)

$$H - Q \qquad (10)$$

umsetzt, oder

b) eine Verbindung der Formel (10) mit einem Orthocarbonsäureester der Formel (7) oder einem Amidin der Formel (8) oder einem Salz eines Amidins der Formel (8) mit einer organischen oder anorganischen Säure in Gegenwart eines Zink, Cadmium, Mangan, Kobalt, Eisen, Kupfer oder Nickel oder ein Gemisch dieser Metalle abgebenden Mittels kondensiert und das erhaltene Kondensationsprodukt mit einem Hydrazon der Formel (6) umsetzt, wobei für A, R, Y und Q das unter Formel (1) Angegebene gilt, $R_2$ eine Alkyl-, Aralkyl- oder Arylgruppe, $R_2'$ ein Wasserstoffatom, eine Alkyl- oder Arylgruppe und Z eine Gruppe $-OR_2$ oder $-NHR_2'$ mit der oben angegebenen Bedeutung von $R_2$ und $R_2'$ darstellen.

Die Herstellung nach Verfahrensvariante a) ist bevorzugt. Die Formel (1) stellt nur eine der möglichen isomeren oder tautomeren Formen dar.

Die Isoindolinonazine der Formel (1) können als Substituenten im Benzolrest A Halogenatome, beispielsweise 2-4 Chloratome, 1-2 Alkyl- oder Alkoxygruppen mit 1-4 C-Atomen, eine Phenyl-, Phenoxy-, Nitro- oder Benzoylaminogruppe, oder eine Alkanoylaminogruppe mit 2-6 C enthalten, sind jedoch vorzugsweise im Rest A unsubstituiert.

R steht beispielsweise für einen Phenyl- oder Naphthylrest, vorzugsweise aber für ein H-Atom oder eine Alkylgruppe mit 1-4 C, insbesondere die Methylgruppe.

In der Verbindung H-Q stellt Q beispielsweise einen Rest der Formel (2) dar, worin $R_1$ vorzugsweise eine Hydroxygruppe und B einen Phenylenrest und vorzugsweise einen Naphthalinrest oder einen 5- oder 6gliedrigen Heteroring bedeuten, der in β-Stellung zum C*-Atom ein O-, S- oder insbesondere ein N-Atom aufweist und gegebenenfalls noch ein weiteres N-Atom im Ring und gegebenenfalls einen ankondensierten Benzolring und/oder einen weiteren Heteroring enthält.

Als isocyclische Hydroxyverbindungen seien solche der Formel (11)

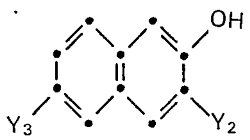

(11)

genannt, worin $R_2$ ein H-Atom, eine Carboxy- oder Carbamoylgruppe, eine Alkoxycarbonyl- oder Alkylcarbamoylgruppe enthaltend 2-6 C-Atome, eine gegebenenfalls im Phenylrest durch Halogenatome, Alkyl- oder Alkoxygruppen, enthaltend 1-4 C-Atome substituierte Phenylcarbamoylgruppe und $Y_3$ ein H- oder Halogenatom, eine Methoxy-, Nitro- oder Cyangruppe bedeuten. Als Beispiele isocyclischer Hydroxyverbindungen seien genannt: 2-Naphthol, 6-Brom-2-naphthol, 6-Nitro-2-naphthol, 2,3-Hydroxynaphthoesäure, 2-Hydroxy-3-naphthoesäureanilid, 2-Hydroxy-6-brom-3-naphthoesäureanilid. Als Beispiel einer cycloaliphatischen methylenaktiven Verbindung wird 5,5-Dimethylcyclohexandion-1,3 (Dimedon) genannt.

Bevorzugt sind heterocyclische Hydroxyverbindungen, beispielsweise Verbindungen der Formeln (12), (13) und (14)

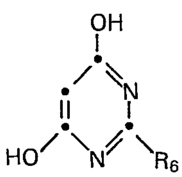

(12)

,

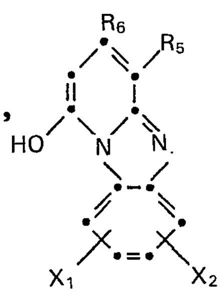

(13)

und

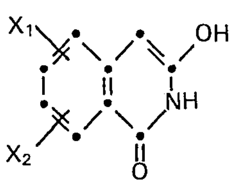

(14)

und insbesondere Verbindungen der Formeln (15) oder (16)

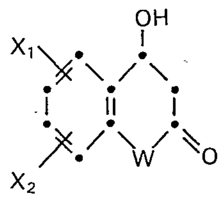

(15)

oder

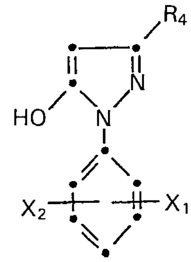

(16),

worin

$X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe,

$X_2$ ein Wasserstoff-, Chlor- oder Bromatom oder eine Alkyl- oder Alkoxygruppe mit 1-4 C,

W O, S oder NH,

$R_4$ Alkyl mit 1-4 C, Alkoxycarbonyl mit 2-6 C oder Carbamoyl,

$R_5$ eine Cyangruppe, eine Alkoxycarbonylgruppe mit 2-6 C oder eine Carbamoylgruppe und

$R_6$ ein Wasserstoffatom, eine Alkylgruppe mit 1-4 C, eine Phenyl- oder Hydroxygruppe bedeuten.

Als Beispiele von heterocyclischen Hydroxyverbindungen H-Q seien genannt: 2,4-Dihydroxychinolin, 2,4-Dihydroxy-5-, -6-, -7-, oder -8-chlorchino-2,4-Dihydroxy-6-, -7- oder -8-methylchinolin, 2,4-Dihydroxy-6-chlor-8-methylchinolin, 2-Methyl-4-hydroxychinolin, 2-Methyl-4-hydroxy-6-chlorchinolin, 2-Methyl-4-hydroxy-6-methoxychinolin, 3-Hydroxyisochinolin, Barbitursäure, 2-Methyl-4,6-dihydroxypyrimidin, 2-Hydroxycumarin, 4-Hydroxy-6-methylcumarin, 4-Hydroxy-6-methoxycumarin, 4-Hydroxy-6-chlorcumarin, 4-Hydroxy-6-chlor-5,7-dimethylcumarin, 1-Phenyl-3-methylpyrazolon-5, 1-Phenyl-3-carboxypyrazolon-5, 1-Phenyl-3-carbamoylpyrazolon-5, 1-Phenyl-3-methoxycarbonylpyrazolon-5, 1-Phenyl-3-äthoxycarbonylpyrazolon-5, 1-o-Chlorphenyl-3-methylpyrazolon-5, 1-p-Chlorphenyl-3-methylpyrazolon-5, 1-o-Methylphenyl-3-methylpyrazolon-5 und 1-p-Methylphenyl-3-methylpyrazolon-5.

Stellt Q einen Rest der Formel (3) dar, worin E und V Acylgruppen sind, so handelt es sich vorzugsweise um Acetyl- oder Benzoylgruppen. Bedeuten E und V gegebenenfalls substituierte Carbamoyl- oder Thiocarbamoylgruppen, dann vorzugsweise Alkylcarbamoyl- oder Alkylthiocarbamoylgruppen mit 2-6 C, Benzylcarbamoylgruppen oder eine Gruppe der Formel (17)

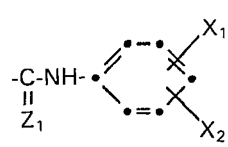

(17)

worin $Z_1$, $X_1$ und $X_2$ die oben angegebene Bedeutung haben. Bedeutet E einen 5- bis 6gliedrigen, gegebenenfalls annellierten Heteroring, dann insbesondere einen solchen, der ein zum C*-Atom β-ständiges N-Atom und N, O oder S als weiteres Heteroatom enthält. Zur Einführung eines Restes Q der Formel (3) verwendet man vorzugsweise Verbindungen der Formel (18)

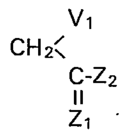
(18)

worin $V_1$ eine Acetyl-, Benzoyl-, Cyan- oder Carbamoylgruppe, eine Alkylcarbamoylgruppe mit 2-6 C oder eine Gruppe der Formel (19)

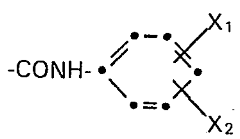
(19)

und $Z_2$ einen Phenylrest oder eine Gruppe der Formel (20)

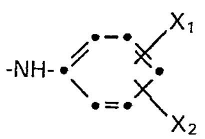
(20)

darstellen und $Z_1$, $X_1$ und $X_2$ die oben angegebene Bedeutung haben.

Als Beispiele von Verbindungen zur Einführung von Resten Q der Formel (3) seien die in der GB-PS 1 467 595 auf Seite 7 aufgeführten substituierten Acetonitrile genannt; ferner Acetoacetylanilin, Acetoacetyl-p-chloranilin, Acetoacetyl-o-methoxyanilin, Acetoacetyl-2,5-dimethoxy-4-chloranilin, 2-Acetoacetylaminobenzimidazol, Thiocarbamoylacetonitril, N-Phenylthiocarbamoylacetonitril, N-p--Chlorphenylthiocarbamoylacetonitril, N-p-Tolylthiocarbamoylacetonitril, N-p-Acetylaminophenylthiocarbamoylacetonitril, N-m-Trifluormethylphenylthiocarbamoylacetonitril, N-p-Nitrophenylthiocarbamoylacetonitril, N-α-Naphthylthiocarbamoylacetonitril, N-p-Methoxyphenylthiocarbamoylacetonitril, Thioacetylaceton, Thioacetessigsäureanilid, N-Cyclohexylthiocarbamoylacetonitril, Cyanacetamid, Cyanessigsäure-N-methylamid, Cyanessigsäure-p-chloranilid, Acetylacetophenon, Cyanacetophenon, 2-Cyanmethylbenzimidazol, 2-Cyanmethyl-thiazolon-4.

Sofern Q für einen Rest der Formel (4) steht, kann $H_2N$-T entweder ein heterocyclisches Amin oder ein Thiocarbamoylderivat sein. Als heterocyclische Amine der Formel $H_2N$-T sind solche bevorzugt, deren Aminogruppe an einem 5- bis 6gliedrigen Heteroring sitzt, der ein zur Aminogruppe β-ständiges N-Atom und gegebenenfalls N, O oder S als weiteres Heteroatom enthält und durch einen Benzolring annelliert sein kann. Als Beispiele seien die auf Seiten 6-7 der GB-PS 1 467 595 aufgeführten Amine, ferner 3-Amino-isoindoleninon-1, 1-Amino-4-chlorphthalazin oder 1,4-Diamino-phthalazin bzw. deren Iminformen genannt. Als heterocyclische Amine verwendet man vorzugsweise solche der Formel (21)

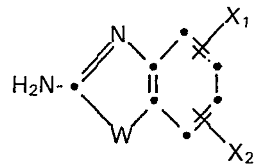
(21)

worin W, $X_1$ und $X_2$ die oben angegebene Bedeutung haben.

Als Thiocarbamoylderivate der Formel $H_2N$-T werden solche der Formel (22)

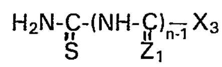
(22)

bevorzugt, worin $X_3$ eine gegebenenfalls durch einen Heteroarylrest oder eine Gruppe der Formel

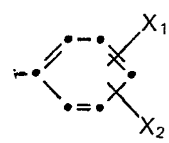

substituierte Aminogruppe bedeutet und $Z_1$, n, $X_1$ und $X_2$ die oben angegebene Bedeutung haben.

Als Hydrazone der Formel (6) verwendet man bevorzugt solche, worin A keine weiteren Substituenten enthält und Y einen Rest der Formeln (23) oder (24)

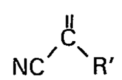
oder
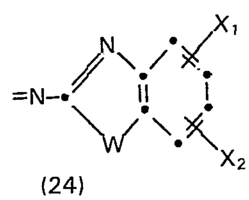

(23) (24)

bedeuten, worin R' für eine Alkoxycarbonyl-, Alkylcarbamoyl-, Carbamoyl-, Thiocarbamoyl- oder Sulfamoylgruppe, eine Benzylcarbamoylgruppe, eine gegebenenfalls durch Halogenatome oder Alkylgruppen mit 1-4 C substituierte Phenylsulfamoyl- oder Phenylsulfonylgruppe, insbesondere aber eine Gruppe der Formeln (17) oder (21a)

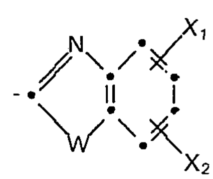
(21a)

steht, worin $Z_1$, $X_1$, $X_2$ und W die oben angegebene Bedeutung haben.

Die als Ausgangstoffe zu verwendenden Hydrazone der Formel (6) stellen bekannte Verbindungen dar, die man beispielsweise nach dem in der GB-PS 1 467 595 beschriebenen Verfahren durch Umsetzung eines Iminoisoindolins der Formel (25)

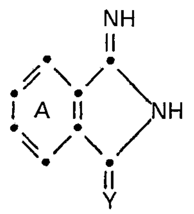

(25)

mit Hydrazinhydrat erhalten kann. Die Verbindungen der Formel (25) ihrerseits erhält man durch Kondensation des entsprechenden 1-Amino-3-iminoisoindolenins mit einem Amin oder einer methylenaktiven Verbindung, insbesondere einer solchen der Formel (26)

$$NCCH_2R' \quad (26)$$

worin R' die oben angegebene Bedeutung hat.

Als Beispiele seien die in der GB-PS 1 467 595, Seite 7, aufgeführten Acetonitrile, ferner Cyanessigsäure-o-chlorphenyl-, -p-chlorphenyl-, -m-chlorphenyl-, -m-methylphenyl-, -p-methylphenyl-, -3,4-dichlorphenyl-, -3,5-dimethylphenyl-, -3,4-dimethylphenyl-, -3-chlor-4-methylphenyl-, -o-methoxyphenyl-, -2,4-dimethoxyphenyl-, -2,5-dimethoxyphenyl-, -p-acetylamino-phenyl-, -p-benzoylaminophenyl-, -3-chlor-4-p-chlorbenzoylaminophenyl-, -4-carbamoylphenyl-, -4-sulfamoylphenyl-, -4-phenylazophenyl-, -4-phenoxyphenyl-, -p-nitrophenyl-, -3-trifluormethylphenyl-, oder -2-chlor-5-trifluormethyl-phenylamid, 2-Cyanmethyl-4-phenyl-, -4-p-nitrophenyl-, -4-fluorphenyl- oder -4-methylphenylthiazol erwähnt. Als methylenaktive Verbindungen kommen auch Heterocyclen, die eine aktive Methylengruppe im Heteroring enthalten, in Betracht, wie sie beispielsweise auf Seite 7 und 8 der GB-PS 1 467 595 erwähnt sind, beispielsweise 2,4-Dihydroxychinolin, 1-p-Chlorphenyl-3-methyl-5-pyrazolon, 1-p-Methylphenyl-3-methyl-5-pyrazolon, 1-Phenyl-3-dichlorvinyl-5-pyrazolon, 1-p-Methylphenyl-3-dichlorvinyl-5-pyrazolon.

Als Rest eines heterocyclischen aromatischen Amins stellt Y vorzugsweise den Rest eines heterocyclischen aromatischen Amins dar, in dem sich die Aminogruppe direkt an einem 5-6-gliedrigen Heteroring befindet, der 1-3 N-Atome und ausserdem noch O- und S-Atome enthalten kann. An den heterocyclischen Stammkern kann ein gegebenenfalls substituierter Benzolkern ankondensiert sein. Als Beispiele seien die in der GB-PS 1 467 595, S. 6-7, aufgeführten Amine genannt, ausserdem 2-Aminopyridin, 2-Amino-5-chlorpyridin, Diaminophthalazin, 2-Amino-4-hydroxychinolin, 2,6-Diaminopyridin, 2-Amino-4,5-dimethylthiazol.

Als Orthocarbonsäureester der Formel (7) oder Amidine der Formel (8) verwendet man vorzugsweise solche, worin R ein Wasserstoffatom oder eine Methylgruppe, $R_2$ Alkyl mit 1-4 C und $R_2'$ ein Wasserstoffatom, Alkyl mit 1-4 C oder Phenyl bedeuten.

Als metallabgebende Mittel verwendet man vorzugsweise Salze der vorerwähnten Metalle oder Metallgemische, insbesondere Salze des Kupfers und vor allem des Nickels. Zweckmässig verwendet man die Formiate, Acetate oder Stearate dieser Metalle.

Die Umsetzungen werden definitionsgemäss in einem polaren organischen Lösungsmittel, insbesondere einem solchen hydrophiler Natur, vorgenommen, beispielsweise einem Amid, wie Dimethylformamid, Formamid, Dimethylacetamid oder N-Methylpyrrolidon, ferner Dimethylsulfoxid, Acetonitril oder einem Alkohol, wie beispielsweise Äthylcellosolve. Es kann auch ein Gemisch polarer Lösungsmittel verwendet werden. Die Umsetzung der Hydrazone der Formel (6) mit den Orthocarbonsäureestern erfolgt zweckmässig in einem Überschuss der letzteren. Bevorzugte Lösungsmittel sind Dimethylformamid und N-Methylpyrrolidon. Die Umsetzungstemperatur liegt zweckmässig zwischen 100 und 200°C.

Die Verbindungen der Formel (1), worin Q einen Rest der Formel (3) oder (4) darstellt, sowie die Azine der Formel (9) sind neue Verbindungen und ebenfalls Gegenstand vorliegender Anmeldung. Bevorzugt sind Verbindungen der Formel (1), worin A keine weiteren Substituenten hat und Q einen Rest der Formel (4) mit T = Rest der Formel (5) oder einen Rest der Formel (18) darstellt, und Azine der Formel (9), worin A keine weiteren Substituenten aufweist, R ein Wasserstoffatom oder eine Methylgruppe, Z eine Alkoxygruppe mit 1-4 C und Y einen Rest der Formel (23) oder (24) darstellen.

Die Isolierung des erhaltenen Metall-Komplexes erfolgt wie üblich durch Filtration. Das Nutschgut wird mit Lösungsmittel gut gewaschen. Es wird in ausgezeichneter Ausbeute und Reinheit erhalten und kann ohne weitere Reinigung in feinverteilter Form zum Färben von hochmolekularem organischem Material verwendet werden, z.B. Celluloseäthern und -estern, wie Äthylcellulose, Acetylcellulose, Nitrocellulose, Polyamiden bzw. Polyurethanen oder Polyestern, natürlichen Harzen oder Kunstharzen, z.B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureester, thermoplastischen oder härtbaren Acrylharzen, Gummi, Casein, Silikon und Silikonharzen, einzeln oder in Mischungen. Die erwähnten hochmolekularen Verbindungen können als plastische Massen, Schmelzen oder in Form von Spinnlösungen, Lacken oder Druckfarben vorliegen. Je nach Verwendungszweck erweist es sich als vorteilhaft, die Pigmente als Toner oder in Form von Präparaten zu verwenden.

Das Pigment kann in der Form, wie es in der Synthese anfällt, oder in leicht gemahlener Form eingesetzt werden und liefert dann opake Ausfärbungen. Man kann es aber auch einer intensiven Mahlung unterziehen und erhält dann transparente Ausfärbungen, wie beispielsweise farbstarke Metalleffektlackierungen. Anreibungen der Pigmente in Lacken zeichnen sich durch ein günstiges Fliessverhalten aus. Die erhaltenen Färbungen, beispielsweise in Kunststoffen, Fasern und Lacken, zeichnen sich durch grosse Farbstärke, hohe Farbtonreinheit, gute Dispergierbarkeit, gute Überlackier-, Migrations-, Hitze-, Licht- und Wetterechtheit sowie durch einen guten Glanz aus.

In der GB-PS 1 467 595 ist bereits die Herstellung von 1:1-Metallkomplexen von Azinen der Formel

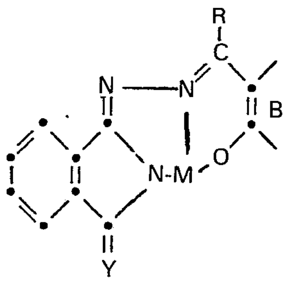

durch Kondensation des entsprechenden Hydrazonoisoindolins mit einer Oxoverbindung der Formel

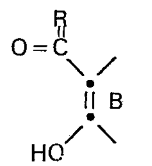

und nachfolgender Metallisierung beschrieben. Demgegenüber bedeutet das erfindungsgemässe Verfahren eine Vereinfachung.

In den nachfolgenden Beispielen bedeuten die Prozente Gewichtsprozente, und die Temperaturen sind in Celsiusgraden angegeben.

*Beispiel 1*

a) 11,6 g 1,3-Diiminoisoindolin 75-proz. (0,06 Mol) und 11,7 g Cyanacet-p-chloranilid (0,06 Mol) werden in 50 ml Dimethylformamid und 3,6 ml (0,06 Mol) Eisessig gelöst und über Nacht bei Raumtemperatur gerührt. Eine dünnschichtchromatographische Kontrolle zeigt am nächsten Tag vollständige Umsetzung zum 1-(Cyano-p-chlorphenylcarbamoylmethylen)-3-imino-isoindolin an. Zu der resultierenden Suspension läst man hierauf bei Raumemperatur innert 1-2 Minuten 3,1 ml Hydrazinhydrat (0,06 Mol) zutropfen. Das Gemisch wird bei derselben Temperatur während 45 Minuten gerührt. Durch anschliessende dünnschichtchromatographische Untersuchung lässt sich vergewissern, dass sich das 1-(Cyano-p-chlor-phenylcarbamoylmethylen)-3-imino-isoindolin vollständig umgesetzt hat, worauf man das Gemisch mit 250 ml Äthanol versetzt und bei Raumtemperatur 15-20 Minuten gut rührt. Die Fällung wird filtriert, mit wenig Äthanol gewaschen und über Nacht bei 50-60°C unter Vakuum getrocknet. Man erhält 16,8 g (83% d. Theorie) der Verbindung der Formel

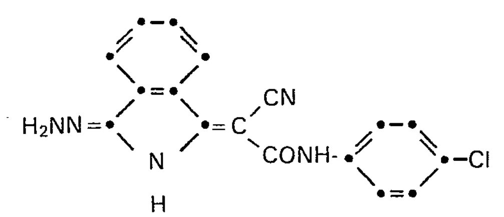

als gelbbraunes Pulver.

b) 25 ml Orthoameisensäuretriäthylester werden mit einem Tropfen konzentrierter Schwefelsäure versetzt und auf 140°C erhitzt. Man gibt unter Rühren bei derselben Temperatur portionenweise 3,4 g (0,01 Mol) der nach Beispiel 1a) erhaltenen Verbindung hinzu, lässt das Gemisch 1 Stunde lang bei 140°C ausreagieren und destilliert zugleich den entstandenen Alkohol ab. Anschliessend wird auf 80°C abgekühlt und filtriert. Das Nutschgut wird mit Sprit gewaschen und über Nacht unter Vakuum bei 80°C getrocknet. Man erhält 3,54 g (90% d. Theorie) der Verbindung der Formel

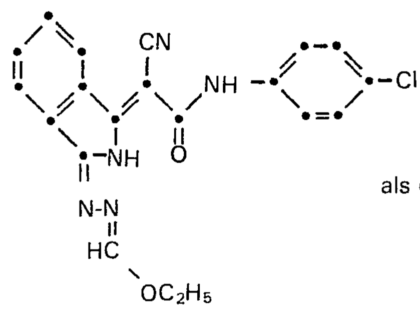

als oranges Pulver.

Mikroanalyse: $C_{20}H_{16}N_5O_2Cl$ MG 393,83

| | | | | |
|---|---|---|---|---|
| gef.: | C 61,00 | H 4,10 | N 17,79 | Cl 9,00 |
| ber.: | C 61,00 | H 4,3 | N 17,8 | Cl 9,0 |

*Beispiel 2*

Zu 40 ml Orthoessigsäuretriäthylester gibt man 1 Tropfen konz. Schwefelsäure und erwärmt auf 130 bis 135°C. Anschliessend trägt man bei derselben Temperatur portionenweise innert 30 Minuten 6,75 g (0,02 Mol) der nach Beispiel 1a) hergestellten Verbindung ein. Das Gemisch lässt man 1 h bei 140°C ausreagieren. Es wird dann auf 80°C abgekühlt und filtriert. Nach üblichem Waschen mit Alkohol und Trocknen unter Vakuum bei 80°C erhält man 6,95 g (85% d. Theorie) der Verbindung der Formel

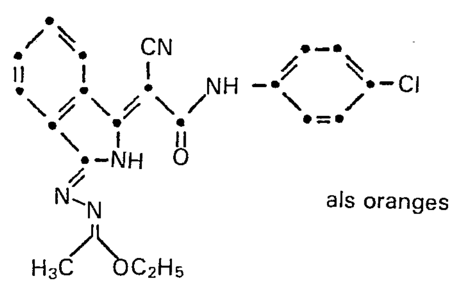

als oranges Pulver.

Mikroanalyse: $C_{21}H_{18}N_5O_2Cl$ MG 407,86

| | | | | |
|---|---|---|---|---|
| ber.: | C 61,84 | H 4,45 | N 17,17 | Cl 8,69 |
| gef.: | C 61,4 | H 4,2 | N 17,8 | Cl 9,1 |

Nach dem Verfahren von Beispiel 1 bzw. 2 werden durch Umsetzung eines Hydrazons der Formel

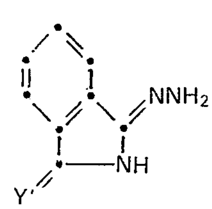

mit einem Orthocarbonsäureester der Formel

$R_1' - C(OR_3'')_3$

Verbindungen der Formel

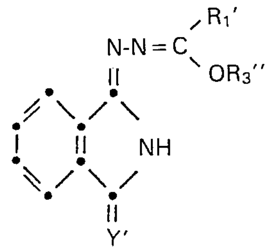

hergestellt, wobei Y', $R_1'$ und $R_3''$ die in der Tabelle 1 angegebene Bedeutung haben.

Tabelle I

| Beispiel Nr. | Y' | $R_1'$ | $R_3''$ |
|---|---|---|---|
| 3 | 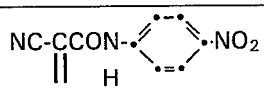 | -H | $-C_2H_5$ |
| 4 | 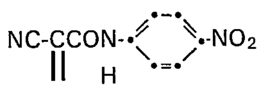 | $CH_3$ | $-C_2H_5$ |
| 5 | 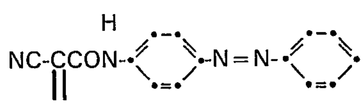 | -H | $-CH_3$ |
| 6 | 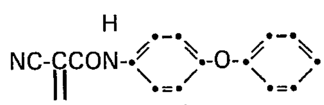 | -H | $-CH_3$ |
| 7 | 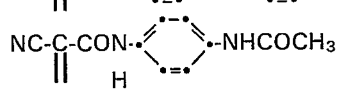 | -H | $-C_2H_5$ |
| 8 | 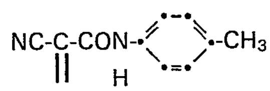 | $-CH_3$ | $-C_2H_5$ |
| 9 | 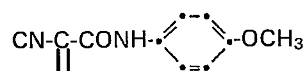 | $-CH_3$ | $-CH_3$ |
| 10 | 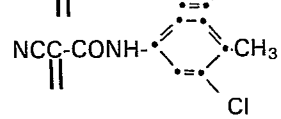 | -H | $-C_2H_5$ |
| 11 | 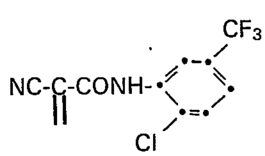 | -H | $-C_2H_5$ |
| 12 | 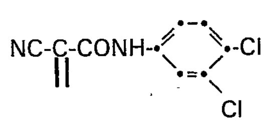 | $-CH_3$ | $-C_2H_5$ |
| 13 | 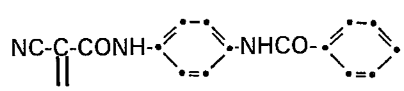 | -H | $-C_2H_5$ |

Tabelle I (Fortsetzung)

| Beispiel Nr. | Y' | $R_1'$ | $R_3''$ |
|---|---|---|---|
| 14 | 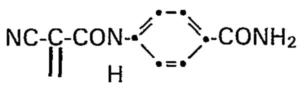 | $-CH_3$ | $-C_2H_5$ |
| 15 | 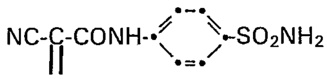 | $-CH_3$ | $-C_2H_5$ |
| 16 | 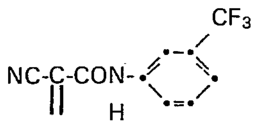 | $-CH_3$ | $-C_2H_5$ |
| 17 | 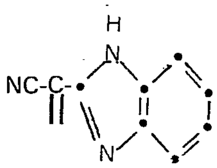 | -H | $-C_2H_5$ |
| 18 | 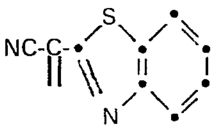 | -H | $-CH_3$ |
| 19 | 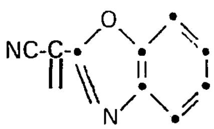 | -H | $-C_2H_5$ |
| 20 | 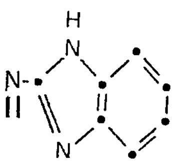 | -H | $-C_2H_5$ |
| 21 | 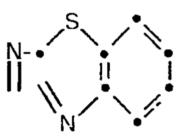 | -H | $-C_2H_5$ |
| 22 | 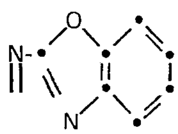 | -H | $-C_2H_5$ |
| 23 | 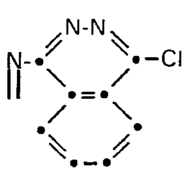 | -H | $-C_2H_5$ |

Tabelle I (Fortsetzung)

| Beispiel Nr. | Y' | $R_1'$ | $R_3''$ |
|---|---|---|---|
| 24 | 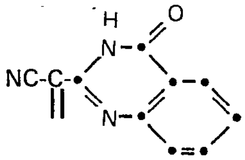 | -H | $-C_2H_5$ |
| 25 | 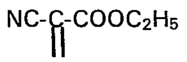 | -H | $-C_2H_5$ |
| 26 | 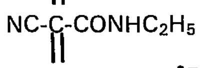 | -H | $-C_2H_5$ |
| 27 | 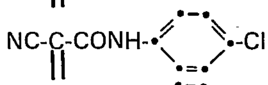 | -H | 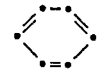 |
| 28 | 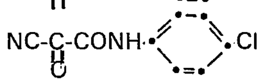 | -H | $-C_4H_9$ |
| 29 | 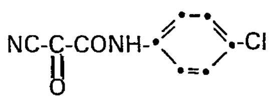 | -H | 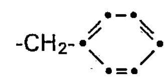 |

*Beispiel 30*

1,97 g (0,005 Mol) der nach Beispiel 1b) erhaltenen Verbindung und 1,31 g (0,0052 Mol) Nickelacetat · $4H_2O$ werden in 35 ml Dimethylformamid angeschlämmt, und das Gemisch auf 60°C erwärmt. Man gibt 1,05 g 1-p-Chlorphenyl-3-methyl-5-pyrazolon (0,005 Mol) hinzu, erhitzt weiter auf 115°C und lässt das Reaktionsgemisch 1 1/2 h bei derselben Temperatur ausreagieren. Nach dem Abkühlen auf 80°C wird filtriert und das Filtergut mit Dimethylformamid und Sprit nachgewaschen und unter Vakuum bei 80°C nachgetrocknet. Man erhält 2,3 g (75% der Theorie) des 1:1-Nickelkomplexes der Formel

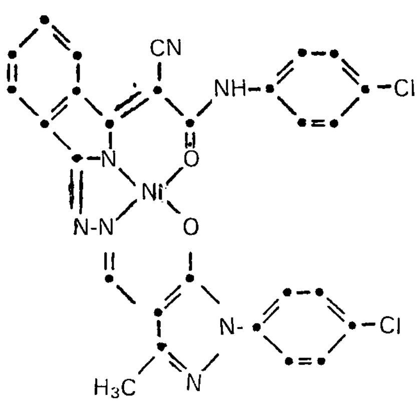

Mikroanalyse: $C_{28}H_{17}Cl_2N_7O_2Ni$ MG 613,1
ber.: C 54,80 H 2,77 Cl 11,58 N 15,98 Ni 9,57
gef.: C 54,5 H 2,9 Cl 11,4 N 16,2 Ni 9,8

Das obige Metallkomplex-Pigment färbt Kunststoffe in reinen scharlachroten Tönen von ausgezeichneten Echtheiten.

*Beispiel 31*

Verfährt man analog Beispiel 30 und verwendet unter sonst gleichen Bedingungen anstelle von 1-p-Chlorphenyl-3-methyl-5-pyrazolon 5,5-Dimethyl-1,3-cyclohexandion, so erhält man nach entsprechender Aufarbeitung einen 1:1-Nickelkomplex (65% der Theorie) der Formel

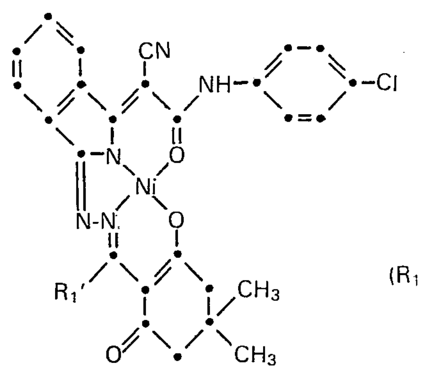

($R_1'$ = H)

Mikroanalyse: $C_{26}H_{20}Cl\,N_5O_3Ni$ MG 544,6
ber.: C 57,34 H 3,7 Cl 12,86 N 6,5 Ni 10,78
gef.: C 57,1 H 3,8 Cl 13,1 N 6,4 Ni 10,9

Das obige Ni-Komplex-Pigment färbt Kunststoffe in reinen orangen Tönen von ausgezeichneter Echtheit.

*Beispiel 32*

Verwendet man im obigen Beispiel 31 anstelle der nach Beispiel 1b) erhaltenen die nach Beispiel 2 erhaltene Verbindung, so erhält man einen ebenfalls reinen orangen Metallkomplex der Formel des Beispiels 31 ($R_1'$ = $CH_3$).

*Beispiel 33*

3,03 g (0,01 Mol) der Verbindung der Formel

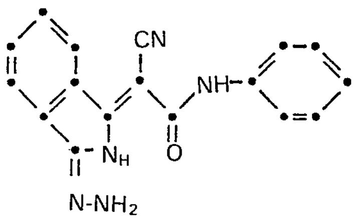
,

hergestellt aus Hydrazinhydrat und 1-(Cyan-phenylcarbamoylmethylen)-3-imino-isoindolin, werden in 40 ml Dimethylformamid angeschlämmt. Man gibt 2 ml (0,012 Mol) Orthoameisensäuretriäthylester hinzu, erwärmt auf 80°C und rührt das Reaktionsgemisch bei derselben Temperatur 10 Minuten lang. Anschliessend versetzt man das Gemisch mit einer 80°C warmen Lösung von 2,08 g (0,01 Mol) 1-p-Chlorphenyl-3-methyl-5-pyrazolon und 2,6 g (0,0105 Mol) Nickelacetat · $4H_2O$ in 25 ml DMF und erhitzt es auf 115°C. Nach einer Reaktionszeit von 2-3 Stunden bei dieser Temperatur wird die Mischung auf 80°C abgekühlt und abfiltriert. Der Pigment-Rückstand wird mit Dimethylformamid und Sprit nachgewaschen und über Nacht unter Vakuum bei 80°C getrocknet. Man erhält 4,7 g (81% der Theorie) des 1:1-Nickelkomplexes der Zusammensetzung $C_{28}H_{18}ClN_7O_2Ni$ und der Formel

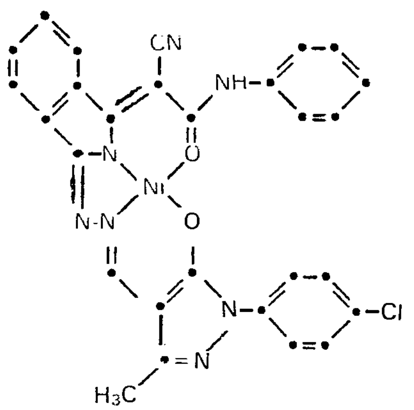

Mikroanalyse: $C_{28}H_{18}ClN_7O_2Ni$ MG 578,66

| | | | | | |
|---|---|---|---|---|---|
| *ber.: | C 57,9 | H 3,14 | N 16,9 | Cl 6,1 | Ni 10,1 |
| gef.: | C 57,4 | H 3,3 | N 17,0 | Cl 6,1 | Ni 10,4 |

* unter Berücksichtigung der gefundenen Wassermenge von 0,3%.

*Beispiel 34*

2,08 g (0,01 Mol) 1-p-Chlorphenyl-3-methyl-5-pyrazolon werden in 40 ml Dimethylformamid angeschlämmt. Nach Zugabe von 2 ml (0,012 Mol) Orthoameisensäuretriäthylester wird das Gemisch auf 80°C erwärmt und 10 Minuten lang bei derselben Temperatur gerührt. Man gibt 2,6 g (0,0105 Mol) Nickelacetat · $4H_2O$ hinzu und rührt weitere 5 Minuten lang bei 80°C. Anschliessend werden 3,03 g (0,01 Mol) des 1-(Cyan-phenylcarbamoylmethylen)-3-hydrazino-isoindolins der Formel

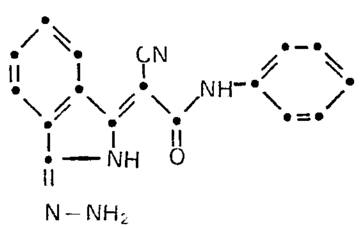

hinzugegeben, das Reaktionsgemisch auf 115°C erhitzt und bei dieser Temperatur 2¹/₂ Stunden gerührt. Nach Abkühlen auf 80°C wird das gebildete Pigment abfiltriert, mit Dimethylformamid und Sprit nachgewaschen und über Nacht bei 80°C unter Vakuum getrocknet. Man erhält 3,05 g (53% der Theorie) eines 1:1-Nickelkomplex-Pigments, das mit dem nach Beispiel 33 erhaltenen Produkt identisch ist.

*Beispiel 35 - 49*

In der Tabelle 2 sind weitere Nickelkomplexe der Formel

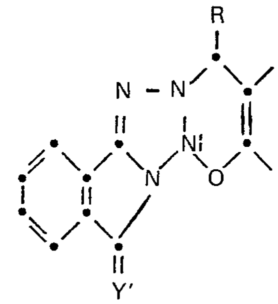
B

aufgeführt (der Einfachheit halber wird nur eine der möglichen isomeren oder tautomeren Formen berücksichtigt), die man erhält, wenn man weitgehend analog Beispiel 30 bzw. 33 entweder das Umsetzungsgemisch eines Hydrazons der Formel

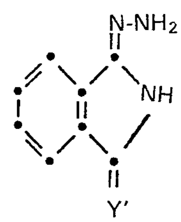

mit Orthoameisensäure- bzw. Orthoessigsäuretriäthylester, oder das Azin der Formel

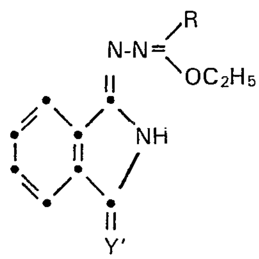

jeweils in Gegenwart von Nickelacetat · $4H_2O$ mit einer methylenaktiven Verbindung der Formel

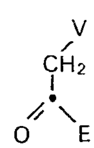

kondensiert, wobei Y', R, V und E die in Kolonnen 2, 3, 4 und 5 angegebene Bedeutung haben. Kolonne 6 gibt die Nuance der Färbung in Polyvinylchlorid wieder.

Tabelle 2

| Beispiel Nr. | Y' | R | V | E | Nuance in PVC |
|---|---|---|---|---|---|
| 35 | 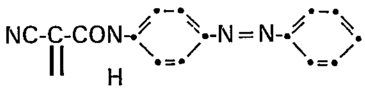 | H | Acetyl | Phenylamino | rot |
| 36 | 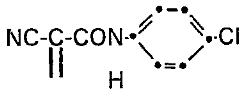 | H | Cyan | p-Chlorphenyl-amino | rot |
| 37 | » | H | P-Methylphe-nylcarbamoyl | p-Methylphenyl-amino | rot |
| 38 | » | H | Acetyl | Phenylamino | rot |
| 39 | » | H | 2-Benzimid-azolyl | Methyl | bordeaux |
| 40 | 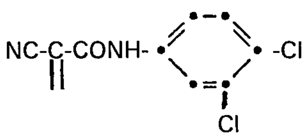 | H | Cyan | Amino | orange |
| 41 | » | H | Cyan | Methylamino | orange |
| 42 | 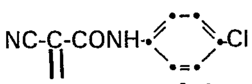 | H | Cyan | Benzylamino | orange |
| 43 | 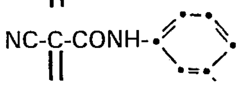 | H | Acetyl | Phenyl | orange |
| 44 | 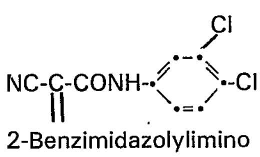 | H | Cyan | Phenyl | orange |
| 45 | 2-Benzimidazolylimino | H | Cyan | p-Chlorphenyl-amino | orange |
| 46 | 2-Benzthiazolylimino | H | Cyan | p-Chlorphenyl-amino | rot |
| 47 | 2-Benzoxazolylimino | H | Cyan | p-Chlorphenyl-amino | orange |
| 48 | 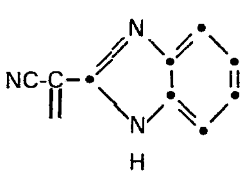 | H | Cyan | phenylamino | rot |
| 49 | 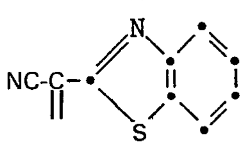 | H | Cyan | phenylamino | rot |

*Beispiel 50*

2,36 g (0,006 Mol) der nach Beispiel 1b) hergestellten Verbindung und 1,57 g (0,0068 Mol) Nickelacetat · $4H_2O$ werden in 40 ml N-Methylpyrrolidon auf ca. 80°C erhitzt. Man gibt 1,06 g (0,006 Mol) Cyanacetthioanilid hinzu und lässt das Gemisch 1 1/2 Stunden lang bei 145°C ausreagieren. Nach Abkühlen auf 80°C wird filtriert, das Nutschgut mit Dimethylformamid und Sprit nachgewaschen und über Nacht bei 80°C unter Vakuum getrocknet. Man erhält 2,22 g (64% der Theorie) eines roten 1:1-Nickelkomplexes der Formel

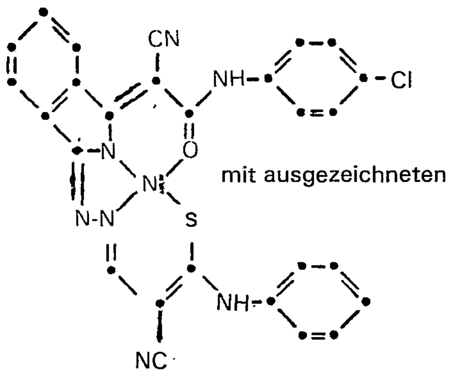

mit ausgezeichneten Echtheiten.

Mikroanalyse: $C_{27}H_{16}ClN_7OSNi$ MG 580,7

ber.:
C 55,85 H 2,78 N 16,88 S 5,52 Cl 6,11 Ni 10,11

gef.:
C 55,1 H 3,1 N 16,4 S 5,5 Cl 5,8 Ni 9,6

*Beispiele 51-55*

In der Tabelle 3 sind weitere Nickelkomplexe der Formel

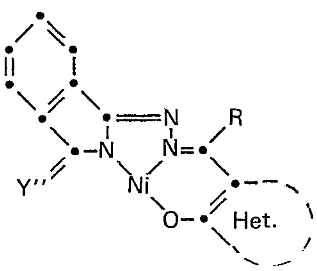

aufgeführt (nur eine der möglichen isomeren oder tautomeren Formen wurde berücksichtigt), die man erhält, wenn man weitgehend analog Beispiel 30 bzw. 33 das Azin der Formel

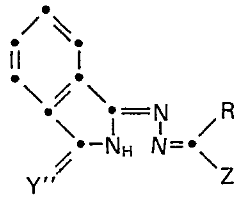

jeweils in Gegenwart von Nickelacetat · $4H_2O$ mit einer heterocyclischen methylenaktiven Verbindung der Formel

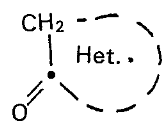

kondensiert, wobei Y'', R, Z und 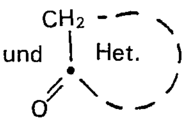 die in Kolonnen 2, 3, 4 und 5 angegebene Bedeutung haben. Kolonne 6 gibt die Nuance der PVC-Färbung.

Tabelle 3

| Beispiel Nr. | Y'' | R | Z | 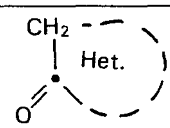 | Nuance in PVC |
|---|---|---|---|---|---|
| 51 | 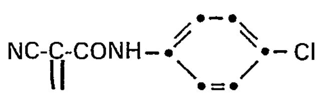 | H | $-NC_3H_7$ H | 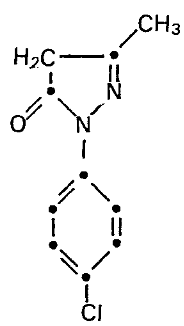 | rot |
| 52 | - do - | H | 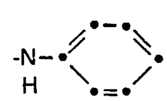 | - do - | rot |
| 53 | - do - | H | $OC_2H_5$ | 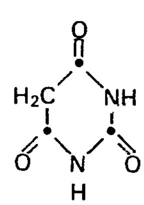 | gelb |

Tabelle 3 (Fortsetzung)

| Beispiel Nr. | Y'' | R | Z | 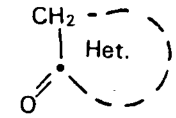 | Nuance in PVC |
| --- | --- | --- | --- | --- | --- |
| 54 | 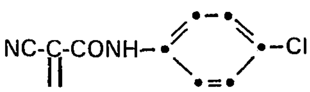 | H | $OC_2H_5$ | 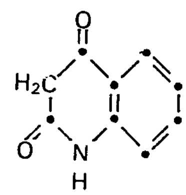 | rot |
| 55 | - do - | H | $OC_2H_5$ | 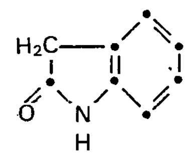 | violett |

*Beispiel 56*

2,36 g (0,006 Mol) der nach Beispiel 1b) erhaltenen Verbindung und 1,57 g (0,0063 Mol) Nickelacetat · $4H_2O$ wurden in 40 ml N-Methylpyrrolidon angeschlämmt und auf 80°C erwärmt. Nach Zugabe von 0,94 g (0,006 Mol) Benzimidazolylacetonitril wird auf 140°C erhitzt und das Gemisch bei derselben Temperatur $1^1/_2$ Stunden lang gerührt. Man lässt anschliessend auf 50°C abkühlen, filtriert, wäscht das Nutschgut mit Dimethylformamid und Sprit und trocknet bei 80°C unter Vakuum. Man erhält 2,2 g (63% der Theorie) eines 1:1-Nickelkomplex-Pigments der Formel

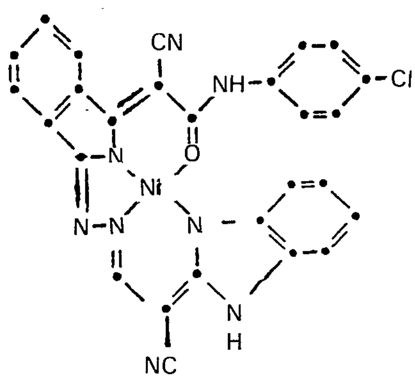

Das Pigment färbt Kunststoffe in bordeaux-roten Tönen von ausgezeichneten Echtheiten.

Mikroanalyse: $C_{27}H_{15}ClN_8ONi$ Mg 561,6
ber.: C 57,74 H 2,69 N 19,95 Cl 6,31 Ni 10,45
gef.: C 57,5 H 3,0 N 20,0 Cl 6,1 Ni 10,6

*Beispiel 57*

1,97 g (0,005 Mol) der nach Beispiel 1b) erhaltenen Verbindung und 1,31 g (0,00525 Mol) Nickelacetat werden in 40 ml N-Methylpyrrolidon angeschlämmt und auf 80°C erwärmt. Man gibt 0,73 g (0,005 Mol) 1-Imino-3-isoindolinon hinzu und lässt das Reaktionsgemisch $1^1/_2$ Stunden lang bei 145°C ausreagieren. Anschliessend kühlt man auf 80°C und filtriert. Das Nutschgut wird mit Dimethylformamid und Sprit nachgewaschen und über Nacht unter Vakuum bei 80°C getrocknet. Man erhält 1,62 g (63% der Theorie) eines 1:1-Nickelkomplexes der Formel

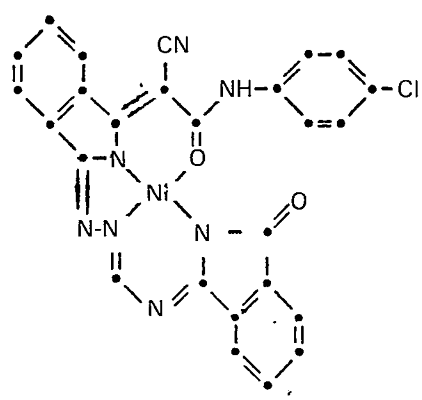

Mikroanalyse: $C_{26}H_{14}ClN_7O_2Ni$ MG 515,16
ber.: C 56,72 H 2,56 N 17,81 Cl 6,44 Ni 10,66
gef.: C 56,0 H 2,7 N 17,5 Cl 6,4 Ni 10,9

Der obige Komplex färbt Kunststoffe in reinen scharlachroten Tönen von ausgezeichneten Echtheiten.

*Beispiel 58-66*

In der Tabelle 4 sind weitere Nickelkomplexe der Formel

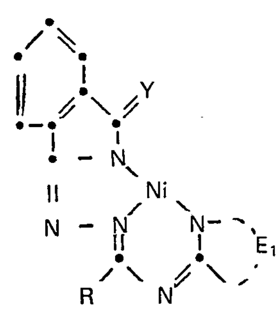

aufgeführt (der Einfachheit halber wird nur eine der möglichen isomeren oder tautomeren Formen berücksichtigt), die man erhält, wenn man analog Beispiel 57 ein Azin der Formel

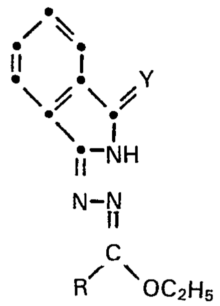

in Gegenwart von Nickelacetat · $4H_2O$ mit einem Amin der Formel $R'NH_2$ kondensiert, wobei Y, R' und R die in Kolonnen 2, 3 und 4 angegebene Bedeutung haben. Kolonne 5 gibt die Nuance der Färbung in Polyvinylchlorid wieder.

Tabelle 4

| Beispiel Nr. | Y | $R'NH_2$ | R | Nuance in PVC |
|---|---|---|---|---|
| 58 | 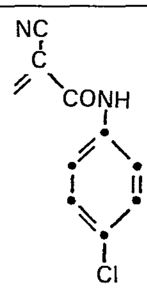 | 1-Amino-4-chlor-phthalazin | H | rot |
| 59 | » | 2-Benzimidazolylimino | H | rot |
| 60 | » | » | $CH_3$ | rot |
| 61 | » | 3-Amino-indazol | H | rot |
| 62 | » | 3-Aminoisoindoleninon (3-Iminoisoindolinon) | H | rot |
| 63 | » | 2-Aminobenzimidazol | $CH_3$ | rot |
| 64 | » | 1,4-Diaminophthalazin | H | orange |
| 65 | 2-Benzthiazolylimino | 3-Amino-isoindoleninon | H | orange |
| 66 | 2-Amino-benzimidazol | 2-Aminobenzimidazol | H | orange |

*Beispiel 67*

2,2 g (0,011 Mol) N,N'-Diphenylformamidin und 3,4 g (0,01 Mol) der nach Beispiel 1a) erhaltenen Verbindung werden in 25 ml Dimethylformamid angeschlämmt. Das Gemisch wird auf 135-140°C erhitzt und 1 h bei derselben Temperatur gerührt. Anschliessend wird es auf Raumtemperatur abgekühlt, mit 80 ml Sprit versetzt und 15 Minuten lang bei Raumtemperatur gut gerührt. Die Fällung wird filtriert, mit wenig Sprit gewaschen und über Nacht bei 50-60°C unter Vakuum getrocknet. Man erhält 3,3 g (75% der Theorie) der Verbindung der Formel

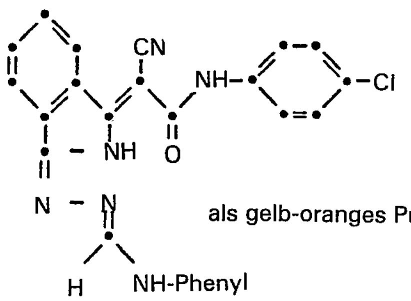

als gelb-oranges Pulver.

Mikroanalyse: $C_{24}H_{17}N_6OCl$ MG 440,5

| | | | | |
|---|---|---|---|---|
| gef.: | C 65,6 | H 3,9 | N 19,2 | Cl 7,9 |
| ber.: | C 65,38 | H 3,85 | N 19,06 | Cl 8,05 |

*Beispiel 68*

Verwendet man in Beispiel 67 anstelle von N,N'-Diphenylformamidin N,N'-Di-n-propylformamidin, so erhält man bei analoger Versuchsdurchführung und Aufarbeitung die folgende Verbindung

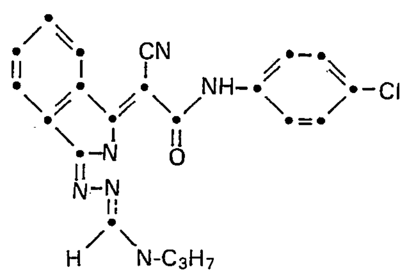

*Beispiel 69*

Verwendet man im Beispiel 33 anstelle des Orthoameisensäuretriäthylesters die entsprechende Menge N,N'-Dipropylformamidin, so erhält man bei analoger Versuchsdurchführung den gleichen roten 1:1-Nickelkomplex wie im Beispiel 33.

*Beispiel 70*

1,25 g (0,005 Mol) Nickelacetat · $4H_2O$ und 1,96 g (0,005 Mol) der nach Beispiel 1b) erhaltenen Verbindung werden in 40 ml Dimethylformamid angeschlämmt und auf 115°C-120°c erwärmt. Nach 5 Minuten Reaktionszeit bei derselben Temperatur wird das Gemisch auf 40°C abgekühlt und mit 0,69 g (0,005 Mol) Thiobenzamid versetzt und langsam auf 100-105°C erhitzt. Man lässt es bei derselben Temperatur 1 Stunde lang ausreagieren, kühlt anschliessend auf 70°C und filtriert. Das Nutschgut wird mit Dimethylformamid und Äthanol gewaschen und über Nacht bei 80°C unter Vakuum getrocknet. Man erhält 1,7 g (61% der Theorie) der Verbindung der Formel

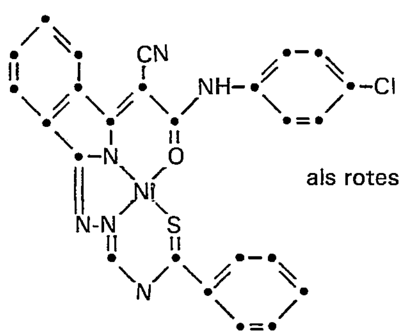

als rotes Pulver.

Mikroanalyse: $C_{25}H_{15}ClN_6OSN$ MG 541,6

| | | | | | | |
|---|---|---|---|---|---|---|
| ber.: | | | | | | |
| | C 55,44 | H 2,79 | N 15,52 | S 5,92 | Cl 6,55 | Ni 10,84 |
| gef.: | | | | | | |
| | C 55,4 | H 3,1 | N 16,0 | S 5,5 | Cl 6,7 | Ni 10,7 |

Der obige Komplex färbt Kunststoffe und Lacke in roten Tönnen von ausgezeichneten Echtheiten.

*Beispiele 71-83*

In der Tabelle 5 sind weitere Nickelkomplexe der Formel

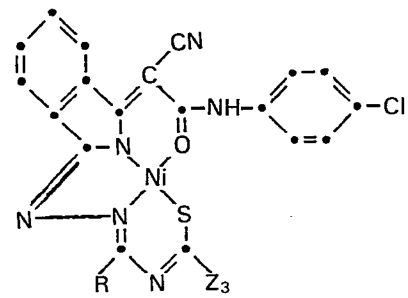

aufgeführt (der Einnfachheit halber wird nur einer der möglichen isomeren oder tautomeren Formen berücksichtigt), die man erhält, wenn man weitgehend analog Beispiel 70 ein Azin der Formel

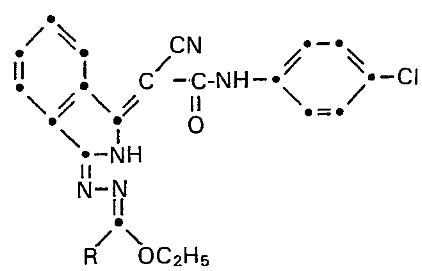

in Gegenwart von Nickelacetat · $4H_2O$ mit einem Thiocarbamoyl-Derivat der Formel

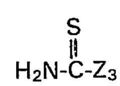

kondensiert, wobei R und $Z_3$ die in Kolonne 2 und 3 angegebene Bedeutung haben. Kolonne 4 gibt die Nuance der Färbung in Polyvinylchlorid wieder.

Tabelle 5

| Beispiel Nr. | R | $Z_3$ | Nuance in PVC |
|---|---|---|---|
| 71 | H | 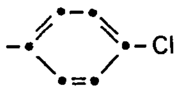 | rot |
| 72 | $CH_3$ | -do- | rot |
| 73 | H | $-CH_3$ | gelb |
| 74 | H | $-NH_2$ | bordeaux |
| 75 | H | 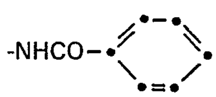 | rot |
| 76 | $CH_3$ | -do- | bordeaux |
| 77 | H | 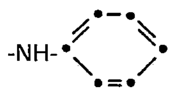 | rot |
| 78 | H | 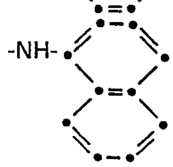 | rot |
| 79 | H | 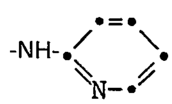 | rot |
| 80 | H | 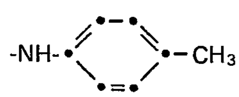 | rot |
| 81 | H | 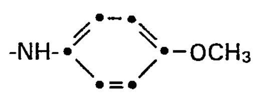 | rot |
| 82 | H | 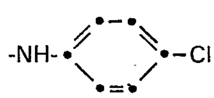 | rot |
| 83 | H | 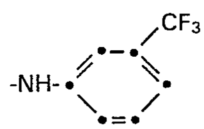 | rot |

*Beispiel 84*

In einem Laboratoriums-Kneter von 250 Vol.-Teilen Inhalt legt man 25 Teile des gemäss Beispiel 30 hergestellten Pigments, 100 Teile feingemahlenes Natriumchlorid und 30 Teile Diacetonalkohol vor. Man knetet die Mischung während 5 Stunden unter Kühlung und trägt sie dann in 4000 Vol.-Teile Wasser ein. Natriumchlorid sowie Diacetonalkohol gehen in Lösung, das Pigment fällt aus. Man filtriert die Suspension, wäscht das Nutschgut gründlich mit Wasser und trocknet es im Vakuumtrockenschrank bei 80°C.

*Beispiel 85*

65 Teile stabilisiertes Polyvinylchlorid, 35 Teile Dioctylphthalat und 0,2 Teile des gemäss Beispiel 56 erhaltenen Pigments werden miteinander verrührt und dann auf einem Zweiwalzenkalander während 7 Minuten bei 140°C hin- und hergewalzt. Man erhält eine orange-rot gefärbte Folie von sehr guter Licht- und Migrationsechtheit.

*Beispiel 86*

10 g Titandioxyd und 2 g des nach Beispiel 56 hergestellten Pigments werden mit 88 g einer Mischung von 26,4 g Kokosalkydharz, 24,0 g Melamin-Formaldehydharz (50% Festkörpergehalt), 8,8 g Äthylenmonomethyläther und 28,8 g Xylol während 48 Stunden in einer Kugelmühle vermahlen. Wird dieser Lack auf eine Aluminiumfolie gespritzt, 30 Minuten bei Raumtemperatur vorgetrocknet und dann während 30 Minuten bei 120°C eingebrannt, dann erhält man eine Rotlackierung, die sich bei guter Farbstärke durch eine sehr gute Überlackier-, Licht- und Wetterechtheit auszeichnet.

*Beispiel 87*

4 Teile des fein verteilten Pigments gemäss Beispiel 56 werden in 20 Teilen Lösungsmittel der folgenden Zusammensetzung eingerührt: 50 Teile Solvesso 150® (Gemisch aromatischer Kohlenstoffe), 15 Teile Butylacetat, 5 Teile Exkin II® (Verlaufmittel auf Ketoximbasis), 25 Teile Methyl-Isobutylketon, 5 Teile Silikonöl (1% in Solvesso 150).

Nachdem die vollständige Feinverteilung erreicht ist (je nach Art des Rührens in ca. 15-60 Minuten), werden die Bindemittel zugesetzt, nämlich 48,3 Teile Baycryl L 530® (Acrylharz) (51% in Xylol/Butanol 3:1) und 23,7 Teile Maprenal TTX® (Melaminharz; 55% in Butanol).

Nach kurzem Homogenisieren wird der Lack nach üblichen Methoden wie Spirtzen und Tauchen oder speziell zur kontinuierlichen Beschichtung von Metallblechen im «Coil-Coating»-Verfahren appliziert und eingebrannt (Einbrennen 30 Minuten, 130°C). Die erhaltenen roten Lackierungen zeichnen sich aus durch sehr guten Verlauf, hohen Glanz und ausgezeichnete Feinverteilung des Pigments, sowie durch ausgezeichnete Wetterechtheiten.

*Beispiel 88*

Verfährt man wie in Beispiel 84 beschrieben, fügt jedoch der Knetmasse 2,78 Teile Staybelite Resin® (HERCULES) zu, so erhält man ein Pigment, enthaltend 10% Harz, das sich auszeichnet durch leichtere Einarbeitbarkeit und bessere Verteilbarkeit.

**Patentansprüche**

1. Verfahren zur Herstellung von 1:1-Metallkomplexen von Isoindolinazinen der Formel (1)

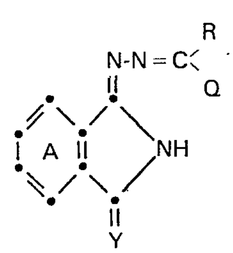

(1)

worin das Metall Zn, Cd, Mn, Co, Fe, Cu oder Ni oder ein Gemisch dieser Metalle ist, der Ring A noch weiter substituiert sein kann,

Y den Rest einer methylenaktiven Verbindung oder eines isocyclischen oder heterocyclischen aromatischen Amins,

R ein Wasserstoffatom, eine Alkyl- oder Arylgruppe und

Q eine Gruppe der Formeln (2), (3) oder (4)

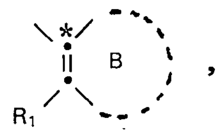 , 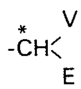 oder -NH-T

(3) (4)

bedeuten, worin

B einen isocyclischen oder heterocyclischen Rest,

$R_1$ eine Hydroxy- oder Mercaptogruppe,

E einen 5- bis 6gliedrigen, ein zum C*-Atom β-ständiges N-Atom enthaltenden und gegebenenfalls annellierten Heteroring, eine Acyl- oder eine gegebenenfalls substituierte Carbamoyl- oder Thiocarbamoylgruppe,

V eine Acyl-, Cyan-, Nitro- oder gegebenenfalls substituierte Carbamoylgruppe und

T einen 5- bis 6gliedrigen, ein zur NH-Gruppe β-ständiges N-Atom enthaltenden und gegebenenfalls annellierten Heteroring oder einen Rest der Formel (5)

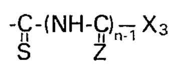 (5)

bedeuten, worin

$Z_1$ für ein O- oder S-Atom, n für die Zahl 1 oder 2 und

$X_3$ für einen Alkyl-, Cycloalkyl-, Aralkyl-, Aryl- oder heterocyclischen Rest oder eine gegebenenfalls durch einen Alkyl-, Cycloalkyl-, Aralkyl-, Aryl- oder Heteroarylrest substituierte Aminogruppe stehen, indem man bei erhöhter Temperatur und in einem polaren organischen Lösungsmittel

a) ein Hydrazon der Formel (6)

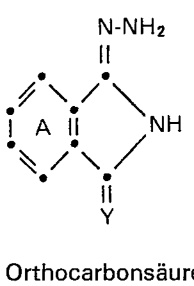 (6)

mit einem Orthocarbonsäureester der Formel (7)

$$C\text{-}R(OR_2)_3 \qquad (7)$$

oder einem Amidin der Formel (8)

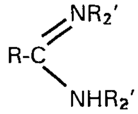 (8)

oder einem Salz eines Amidins der Formel (8) mit einer organischen oder anorganischen Säure zu einem Azin der Formel (9)

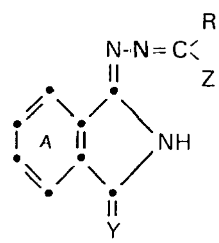 (9)

kondensiert und das erhaltene Kondensationsprodukt in Gegenwart eines Zink, Cadmium, Mangan, Kobalt, Eisen, Kupfer oder Nickel oder ein Gemisch dieser Metalle abgebenden Mittels mit einer Verbindung der Formel (10)

$$H\text{-}Q \qquad (10)$$

umsetzt, oder

b) eine Verbindung der Formel (10) mit einem Orthocarbonsäureester der Formel (7) oder einem Amidin der Formel (8) oder einem Salz eines Amidins der Formel (8) mit einer organischen oder anorganischen Säure in Gegenwart eines Zink, Cadmium, Mangan, Kobalt, Eisen, Kupfer oder Nickel oder ein Gemisch dieser Metalle abgebenden Mittels kondensiert und das erhaltene Kondensationsprodukt mit einem Hydrazon der Formel (6) umsetzt, wobei für A, R, Y und Q das unter Formel (1) Angegebene gilt, $R_2$ eine Alkyl-, Aralkyl- oder Arylgruppe, $R_2'$ ein Wasserstoffatom, eine Alkyl- oder Arylgruppe und Z eine Gruppe $-OR_2$ oder $-NHR_2'$ mit der oben angegebenen Bedeutung von $R_2$ und $R_2'$ darstellen.

2. Verfahren nach Anspruch 1, dadurch gekennzeichnet, dass man als Verbindung der Formel (10) ein solche der Formeln (15) oder (16)

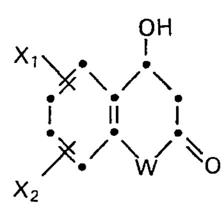 oder 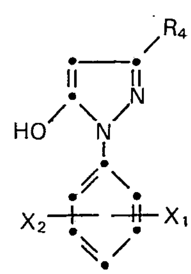

(15) (16),

verwendet, worin W O, S oder NH, $X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein Wasserstoff-, Chlor- oder Bromatom oder eine Alkyl- oder Alkoxygruppe mit 1-4 C und $R_4$ Alkyl mit 1-4 C, Akoxycarbonyl mit 2-6 C oder Carbamoyl bedeuten.

3. Verfahren nach Anspruch 1, dadurch gekennzeichnet, dass man als Verbindung der Formel (10) eine solche der Formel (18)

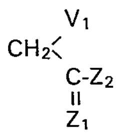

(18)

verwendet, worin $V_1$ eine Acetyl-, Benzoyl-, Cyan- oder Carbamoylgruppe, eine Alkylcarbamoylgruppe mit 2-6 C, eine Benzylcarbamoylgruppe oder eine Gruppe der Formel (19)

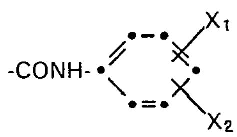

(19)

darstellt, worin $X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein Wasserstoff-, Chlor- oder Bromatom oder eine Alkyl- oder Alkoxygruppe mit 1-4 C, $Z_1$ ein O- oder S-Atom und $Z_2$ einen Phenylrest oder eine Gruppe der Formel (20)

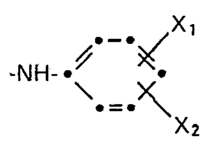

(20)

bedeuten.

4. Verfahren nach Anspruch 1, dadurch gekennzeichnet, dass man als Verbindungen der Formel (10) ein Amin der Formel (21)

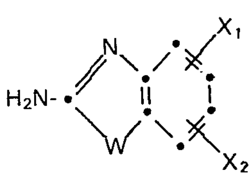

(21)

verwendet, worin W ein O- oder S-Atom oder die NH-Gruppe, $X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe und $X_2$ ein Wasserstoff-, Chlor- oder Bromatom oder eine Alkyl- oder Alkoxygruppe mit 1-4 C bedeuten.

5. Verfahren nach Anspruch 1, dadurch gekennzeichnet, dass man als Verbindung der Formel (10) ein Thiocarbamoylderivat der Formel (22)

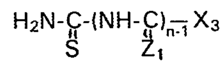

(22)

verwendet, worin n und $Z_1$ die im Anspruch 1 angegebene Bedeutung haben und $X_3$ eine gegebenenfalls durch einen Heteroarylrest oder eine Gruppe der Formel

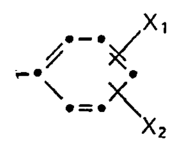

substituierte Aminogruppe bedeutet, worin $X^1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe und $X_2$ ein Wasserstoff-, Chlor- oder Bromatom, oder eine Alkyl- oder Alkoxygruppe mit 1-4 C darstellen.

6. Verfahren nach Anspruch 1, dadurch gekennzeichnet, dass man ein Hydrazon der Formel (6) verwendet, worin A keine weiteren Substituenten enthält und Y einen Rest der Formeln (23) oder (24)

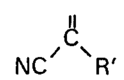 oder 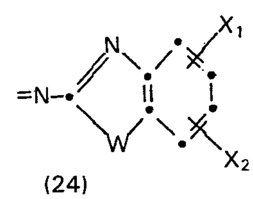

(23) (24)

bedeutet, worin R' für eine Alkoxycarbonyl-, Alkylcarbamoyl-, Carbamoyl-, Thiocarbamoyl- oder Sulfamoylgruppe, eine Benzylcarbamoylgruppe, eine gegebenenfalls durch Halogenatome oder Alkylgruppen mit 1-4 C substituierte Phenylsulfamoyl- oder Phenylsulfonylgruppe, insbesondere aber eine Gruppe der Formeln (17) oder (21a)

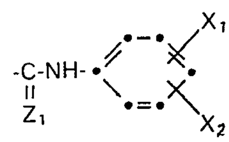

(17)

oder

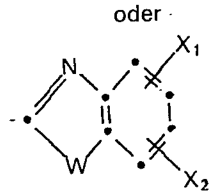

(21a)

steht, worin $Z_1$ die im Anspruch 1 angegebene Bedeutung hat, $X_1$ ein Wasserstoff-, Chlor- oder Bromatom, eine Nitro-, Trifluormethyl-, Carbamoyl- oder

Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein Wasserstoff-, Chlor- oder Bromatom oder eine Alkyl- oder Alkoxygruppe mit 1-4 C und W O, S oder NH bedeuten.

7. Verfahren nach Anspruch 1, dadurch gekennzeichnet, dass man einen Orthocarbonsäureester der Formel (7) oder ein Amidin der Formel (8) verwendet, worin R ein Wasserstoffatom oder eine Methylgruppe, $R_2$ eine Alkylgruppe mit 1-4 C und $R_2'$ ein Wasserstoffatom, Alkyl mit 1-4 C oder eine Phenylgruppe bedeuten.

8. Verfahren nach Anspruch 1, dadurch gekennzeichnet, dass man Salze des Nickels verwendet.

9. Verfahren nach Anspruch 1, dadurch gekennzeichnet, dass man als polares Lösungsmittel Dimethylformamid oder N-Methylpyrrolidon verwendet.

10. Verfahren nach Anspruch 1, dadurch gekennzeichnet, dass man die Umsetzung bei Temperaturen zwischen 100 und 200°C durchführt.

11. Isoindolinverbindungen der Formel (9)

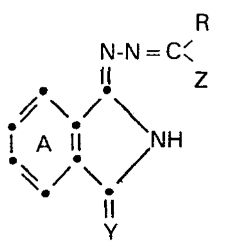

(9)

worin der Ring A noch weiter substituiert sein kann,

Y den Rest einer methylenaktiven Verbindung oder eines isocyclischen oder heterocyclischen aromatischen Amins,

R ein Wasserstoffatom, eine Alkyl- oder Arylgruppe,

Z eine Gruppe $-OR_2$ oder $-NHR_2'$,

$R_2$ eine Alkyl-, Aralkyl- oder Arylgruppe und

$R_2'$ ein Wasserstoffatom, eine Alkyl- oder Arylgruppe bedeuten.

12. Isoindolinverbindungen nach Anspruch 11, worin A keine weiteren Substituenten aufweist, R ein Wasserstoffatom oder eine Methylgruppe, Z eine Alkoxygruppe mit 1-4 C und Y einen Rest der Formeln (23) oder (24)

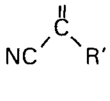

oder

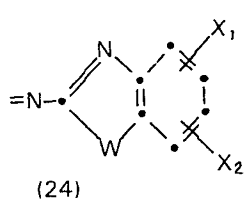

(23) (24)

bedeuten, worin R' für eine Alkoxycarbonyl-, Alkylcarbamoyl-, Carbamoyl-, Thiocarbamoyl- oder Sulfamoylgruppe, eine Benzylcarbamoylgruppe, eine gegebenenfalls durch Halogenatome oder Alkylgruppen mit 1-4 C substituierte Phenylsulfamoyl- oder Phenylsulfonylgruppe, insbesondere aber eine Gruppe der Formeln (17) oder (21a)

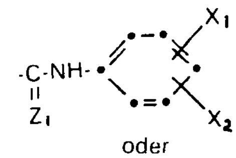

(17)

oder

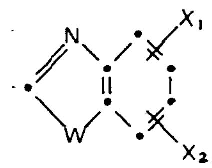

(21a)

steht, $Z_1$ ein O- oder S-Atom, $X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein Wasserstoff-, Chlor oder Bromatom oder eine Alkyl- oder Alkoxygruppe mit 1-4 C und W O, S oder NH bedeuten.

13. 1:1-Metallkomplexe von Azinen der Formel (1)

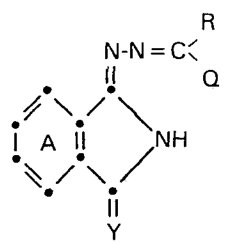

(1)

worin das Metall Zn, Cd, Mn, Co, Fe, Cu oder Ni oder ein Gemisch dieser Metalle ist, der Ring A noch weiter substituiert sein kann,

Y den Rest einer methylenaktiven Verbindung oder eines isocyclischen oder heterocyclischen aromatischen Amins,

R ein Wasserstoffatom, eine Alkyl- oder Arylgruppe und

Q eine Gruppe der Formel (3) oder (4)

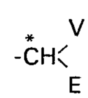

oder -NH-T

(3) (4)

bedeuten, worin

E einen 5- bis 6gliedrigen, ein zum C*-Atom β-ständiges N-Atom enthaltenden und gegebenenfalls annellierten Heteroring, eine Acyl- oder eine gegebenenfalls substituierte Carbamoyl- oder Thiocarbamoylgruppe,

V eine Acyl-, Cyan-, Nitro- oder gegebenenfalls substituierte Carbamoylgruppe und

T einen 5- bis 6gliedrigen, ein zur NH-Gruppe β-ständiges N-Atom enthaltenden und gegebenenfalls annellierten Heteroring oder einen Rest der Formel

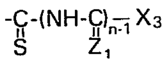 (5)

bedeuten, worin

$Z_1$ für ein O- oder S-Atom, n für die Zahl 1 oder 2 und

$X_3$ für einen Alkyl-, Cycloalkyl-, Aralkyl-, Aryl- oder heterocyclischen Rest oder eine gegebenenfalls durch einen Alkyl-, Cycloalkyl-, Aralkyl-, Aryl- oder Heteroarylrest substituierte Aminogruppe stehen.

14. 1:1-Metallkomplexe von Azinen der Formel (1) nach Anspruch 13, worin A keine weiteren Substituenten hat und Q einen Rest der Formel (18)

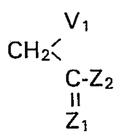 (18)

bedeutet, worin $V_1$ eine Acetyl-, Benzoyl-, Cyan- oder Carbamoylgruppe, eine Alkylcarbamoylgruppe mit 2-6 C, eine Benzylcarbamoylgruppe oder eine Gruppe der Formel (19)

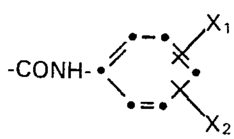 (19)

darstellt, worin $X_1$ ein Wasserstoff-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogrppe, $X_2$ ein Wasserstoff-, Chlor- oder Bromatom oder eine Alkyl- oder Alkoxygruppe mit 1-4 C, $Z_1$ ein O- oder S-Atom und $Z_2$ einen Phenylrest oder eine Gruppe der Formel (20)

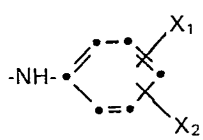 (20)

bedeuten, worin $X_1$ und $X_2$ die angegebene Bedeutung haben.

15. 1:1-Metallkomplexe von Azinen der Formel (1) nach Anspruch 13, worin A keine weiteren Substituenten aufweist und Q einen Rest der Formel

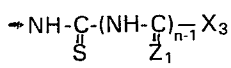

bedeutet, worin n, $X_3$ und $Z_1$ die im Anspruch 13 angegebene Bedeutung haben.

16. Verfahren zum Pigmentieren von hochmolekularem organischem Material, gekennzeichnet durch die Verwendung der Metallkomplexe gemäss Anspruch 13.

17. Hochmolekulares organisches Material enthaltend einen Metallkomplex gemäss Anspruch 13.

**Claims**

1. A process for the manufacture of a 1:1 metal complex of an isoindoline azine of the formula (1)

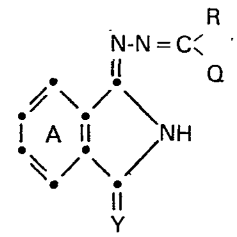 (1)

wherein the metal is Zn, Cd, Mn, Co, Fe, Cu or Ni or a mixture thereof, the ring A can be still further substituted,

Y is the radical of a compound containing active methylene groups or of an isocyclic or heterocyclic aromatic amine,

R is a hydrogen atom, an alkyl or aryl group and

Q is a group of the formula (2), (3) or (4)

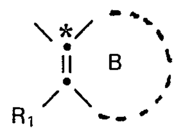 , 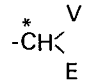 oder -NH-T

(3) (4)

wherein

B is an isocyclic or heterocyclic radical,

$R_1$ is a hydroxyl or mercapto group,

E is a 5- or 6-membered heterocyclic ring which may be fused with benzene nuclei and which contains a nitrogen atom β-oriented to the C* atom, an aryl group, an unsubstituted or a substituted carbamoyl or thiocarbamoyl group,

V is an acyl, cyano or nitro group or an unsubstituted or substituted carbamoyl group,

T is a 5- or 6-membered heterocyclic ring which may be fused with benzene nuclei and which contains a nitrogen atom β-oriented to the NH group, or is a radical of the formula (5)

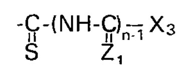 (5)

wherein

$Z_1$ is an oxygen or a sulfur atom, n is 1 or 2,

$X_3$ is an alkyl, cycloalkyl, aralkyl. aryl or heterocyclic radical, or an amino group which is unsubstituted or substituted by an alkyl, cycloalkyl, aralkyl, aryl or heteroaryl radical, which process comprises

a) condensing a hydrazone of the formula (6)

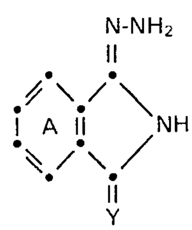 (6)

with an ortho-carboxylic acid ester of the formula (7)

$$R\text{-}C(OR_2)_3 \quad (7)$$

or with an amidine of the formula (8)

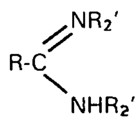

(8)

or with a salt of an amidine of the formula (8) with an organic or inorganic salt, to give an azine of the formula (9)

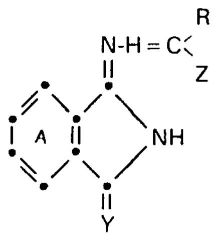

(9)

and reacting the condensation product with a compound of the formula (10)

H - Q (10)

in the presence of a metal donor which donates zinc, cadmium, manganese, cobalt, iron, copper or nickel or a mixture thereof, or

b) condensing a compound of the formula (10) with an orthocarboxylic acid ester of the formula (7) or with an amidine of the formula (8) or with a salt of an amidine of the formula (8) with an organic or inorganic acid, in the presence of a metal donor which donates zinc, cadmium, manganese, cobalt, iron, copper or nickel or a mixture thereof, and reacting the condensation product with a hydrazone of the formula (6), wherein A, R, Y and Q are as defined for formula (1), $R_2$ is an alkyl, aralkyl or aryl group, $R_2'$ is a hydrogen atom, an alkyl or aryl group and Z is an $-OR_2$ or $-NHR_2'$ group, wherein $R_2$ and $R_2'$ are as defined above, the above reactions being carried out at elevated temperature and in a polar organic solvent.

2. A process according to claim 1, wherein the compound of the formula (10) is a compound of the formula (15) or (16)

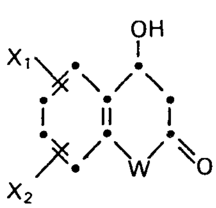

(15)

or

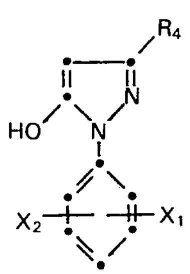

(16) ,

wherein W is O, S or NH, $X_1$ is a hydrogen, chlorine or bromine atom, a nitro, trifluormethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl or phenylsulfamoyl or phenylazo group which is unsubstituted or substituted by chlorine or bromine atoms or methyl groups, and $X_2$ is a hydrogen, chlorine or bromine atom, an alkyl or alkoxy group of 1 to 4 carbon atoms, and $R_4$ is alkyl of 1 to 4 carbon atoms, alkoxycarbonyl of 2 to 6 carbon atoms or carbamoyl.

3. A process according to claim 1, wherein the compound of the formula (10) is a compound of the formula (18)

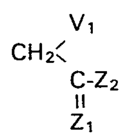

(18)

wherein $V_1$ is an acetyl, benzoyl, cyano or carbamoyl group, an alkylcarbamoyl group containg 2 to 6 carbon atoms, a benzylcarbamoyl group or a group of the formula (19)

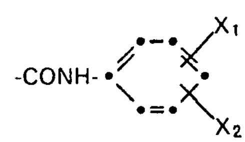

(19)

wherein $X_1$ is a hydrogen, chlorine or bromine atom, a nitro, trifluormethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl or phenylsulfamoyl or phenylazo group which is unsubstituted or substituted by chlorine or bromine atoms or methyl groups, and $X_2$ is a hydrogen, chlorine or bromine atom, an alkyl or alkoxy group of 1 to 4 carbon atoms, $Z_1$ is an oxygen or a sulfur atom, $Z_2$ is a phenyl radical or a group of the formula (20)

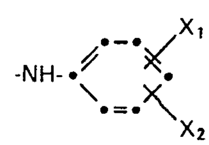

(20)

4. A process according to claim 1, wherein the compound of formula (10) is an amine of the formula (21)

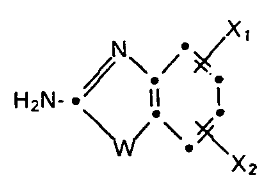

(21)

wherein W is an oxygen or sulfur atom or the -NH group, $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl or phenylsulfamoyl or phenylazo group which is unsubstituted or substituted by chlorine or bromine atoms or methyl groups, and $X_2$ is a hydrogen, chlorine or bromine atom, an alkyl or alkoxy group of 1 to 4 carbon atoms.

5. A process according to claim 1, wherein the compound of formula (10) is a thiocarbamoyl derivative of the formula (22)

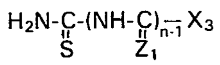 (22)

wherein n and $Z_1$ are as defined in claim 1 and $X_3$ is an amino group which is unsubstituted or substituted by a heteroaryl radical or by a group of the formula

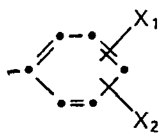

wherein $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl or phenylsulfamoyl or phenylazo group which is unsubstituted or substituted by chlorine or bromine atoms or methyl groups, and $X_2$ is a hydrogen, chlorine or bromine atom, an alkyl or alkoxy group of 1 to 4 carbon atoms.

6. A process according to claim 1 which comprises the use of a hydrazone of the formula (6), wherein A is not further substituted and Y is a radical of the formula (23) or (24)

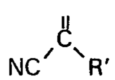 or 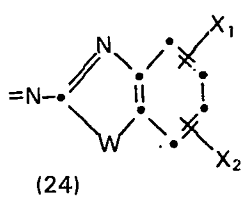

(23) (24)

wherein R' is an alkoxycarbonyl, alkylcarbamoyl, carbamoyl, thiocarbamoyl or sulfamoyl group, a benzylcarbamoyl group, a phenylsulfamoyl or phenylsulfonyl group which is unsubstituted or substituted by halogen atoms or alkyl groups of 1 to 4 carbon atoms, but is especially a group of the formula (17) or (21a)

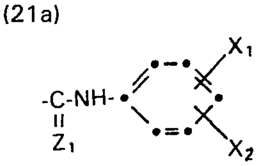 (17)

or 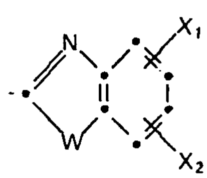 (21a)

wherein $Z_1$ is as defined in claim 1, $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl, phenylsulfamoyl or phenylazo group which is unsubstituted or substituted by chlorine or bromine atoms or methyl groups, and $X_2$ is a hydrogen, chlorine or bromine atom, or an alkyl or alkoxy group of 1 to 4 carbon atoms, and W is O, S, or NH.

7. A process according to claim 1 which comprises the use of an ortho-carboxylic acid ester of the formula (7) or of an amidine of the formula (8), wherein R is a hydrogen atom or a methyl group, $R_2$ is an alkyl group of 1 to 4 carbon atoms and $R_2'$ is a hydrogen atom, an alkyl group of 1 to 4 carbon atoms, or a phenyl group.

8. A process according to claim 1, wherein a nickel salt is used as metal donor.

9. A process according to claim 1, wherein dimethyl formamide or N-methylpyrrolidone is used as polar solvent.

10. A process according to claim 1, wherein the reaction is carried out in the temperature range from 100°-200°C.

11. An isoindoline compound of the formula (9)

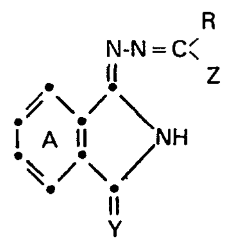 (9)

wherein the ring A may be still further substituted,

Y is the radical of a compound containing active methylene groups or of an isocyclic or heterocyclic aromatic amine,

R is a hydrogen atom, an alkyl or aryl group,

Z is an $-OR_2$ or $-NHR_2'$ group,

$R_2$ is an alkyl, aralkyl or aryl group and

$R_2'$ is a hydrogen atom, or an alkyl or aryl group.

12. An isoindoline compound according to claim 11, wherein A is not further substituted, R is a hydrogen atom or a methyl group, Z is an alkoxy group of 1 to 4 carbon atoms and Y is a radical of the formula (23) or (24)

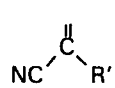 or 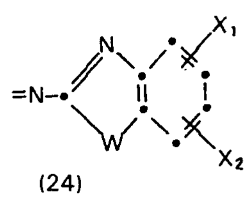

(23) (24)

wherein R' is an alkoxycarbonyl, alkylcarbamoyl, carbamoyl, thiocarbamoyl or sulfamoyl group, a benzylcarbamoyl group, a phenylsulfamoyl or phenylsulfonyl group which is unsubstituted or substituted by halogen atoms or alkyl groups of 1 to 4 carbon atoms, but is especially a group of the formula (17) or (21a)

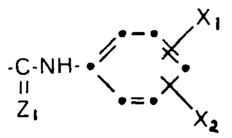

(17)

or

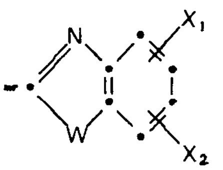

(21a)

wherein $Z_1$ is an oxygen or a sulfur atom, $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl, phenylsulfamoyl or phenylazo group which is unsubstituted or substituted by chlorine or bromine atoms or methyl groups, and $X_2$ is a hydrogen, chlorine or bromine atom, or an alkyl or alkoxy group of 1 to 4 carbon atoms, and W is O, S, or NH.

13. A 1:1 metal complex of an azine of the formula (1)

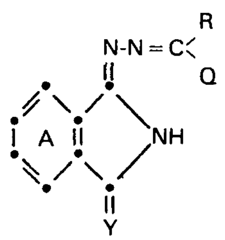

(1)

wherein the metal is Zn, Cd, Mn, Co, Fe, Cu or Ni or a mixture thereof, the ring A can be still further substituted,

Y is the radical of a compound containing active methylene groups or of an isocyclic or heterocyclic aromatic amine,

R is a hydrogen atom, an alkyl or aryl group and

Q is a group of the formula (3) or (4)

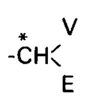

-NH-T

(3) (4)

wherein

E is a 5- or 6-membered heterocyclic ring which may be fused with benzene nuclei and which contains a nitrogen atom $\beta$-otiented to the C* atom, an aryl group, an unsubstituted or a substituted carbamoyl or thiocarbamoyl group,

V is an acyl, cyano or nitro group or an unsubstituted or substituted carbamoyl group,

T is a 5- or 6-membered heterocyclic ring which may be fused with benzene nuclei and which contains a nitrogen atom $\beta$-oriented to the NH group, or is a radical of the formula (5)

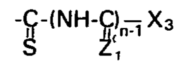

(5)

wherein

$Z_1$ is an oxygen or a sulfur atom, n is 1 or 2,

$X_3$ is an alkyl, cycloalkyl, aralkyl, aryl or heterocyclic radical, or an amino group which is unsubstituted or substituted by an alkyl, cycloalkyl, aralkyl, aryl or heteroaryl radical.

14. A 1:1 metal complex of an azine of the formula (1) according to claim 1, wherein A does not contain further substituents and Q is a radical of the formula (18)

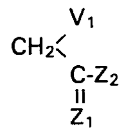

(18)

wherein $V_1$ is an acetyl , benzoyl, cyano or carbamoyl group, an alkylcarbamoyl group of 2 to 6 carbon atoms, a benzylcarbamoyl group or a group of the formula (19)

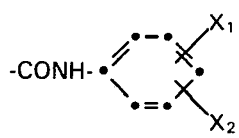

(19)

wherein $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, benzoylamino, phenylcarbamoyl or phenylsulfamoyl or phenylazo group which is unsubstituted or substituted by chlorine or bromine atoms or methyl groups, $X_2$ is a hydrogen, chlorine or bromine atom, or an alkyl or alkoxy group of 1 to 4 carbon atoms, $Z_1$ is an oxygen or sulfur atom and $Z_2$ is a phenyl radical or a group of the formula (20)

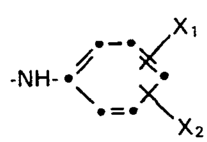

(20)

wherein $X_1$ and $X_2$ have the given meanings.

15. A 1:1 metal complex of an azine of the formula (1) according to claim 13, wherein A is not further substituted and Q is a radical of the formula

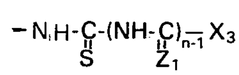

wherein n and $X_3$ and $Z_1$ are as defined in claim 13.

16. A process for pigmenting high molecular organic material, which comprises the use of a metal complex according to claim 13.

17. Organic material of high molecular weight which contains a metal complex according to claim 13.

**Revendications**

1. Procédé de préparation de complexes métalliques 1:1 dont le métal est Zn, Cd, Mn, Co, Fe, Cu ou Ni, ou un mélange de ces métaux, et qui dérivent d'iso-indolinone-azines répondant à la formule (1)

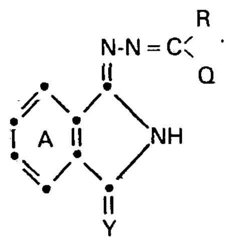

(1)

dans laquelle
le noyau A peut porter d'autres substituants,

Y représente le radical d'un composé à méthylène actif ou d'une amine aromatique isocyclique ou hétérocyclique,

R représente un atome d'hydrogène ou un radical alkyle ou aryle et

Q représente un radical répondant à l'une des formules (2), (3) et (4)

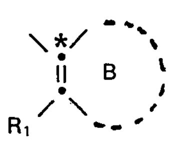

, 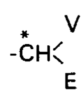 oder -NH-T

(3) (4)

dans lesquelles

B représente un radical isocyclique ou hétérocyclique,

$R_1$ représente un radical hydroxy ou mercapto,

E représente un hétérocycle pentagonal ou hexagonal contenant un atome d'azote en position $\beta$ par rapport à l'atome C* et éventuellement condensé, un radical acyle ou un radical carbamoyle ou thiocarbamoyle éventuellement substitué,

V représente un radical acyle, cyano ou nitro ou un radical carbamoyle éventuellement substitué et

T représente un radical hétérocyclique pentagonal ou hexagonal, contenant un atome d'azote en position $\beta$ par rapport au groupe -NH et éventuellement condensé, ou un radical répondant à la formule (5)

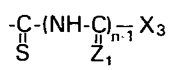

(5)

dans laquelle $Z_1$ représente un atome d'oxygène ou de soufre, n est égal à 1 ou à 2 et $X_3$ représente un radical alkyle, cyclo-alkyle, aralkyle, aryle ou hétérocyclique ou un radical amino éventuellement porteur d'un radical alkyle, cyclo-alkyle, aralkyle, aryle ou hétéroaryle,

procédé selon lequel on condense à température élevée et dans un solvant organique polaire:

a) une hydrazone de formule (6)

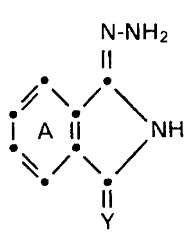

(6)

avec un ester orthocarboxylique de formule (7)

$$R\text{-}C(OR_2)_3 \qquad (7)$$

ou une amidine de formule (8)

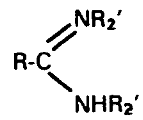

(8)

ou un sel d'une amidine de formule (8) avec un acide minéral ou organique, de manière à obtenir une azine de formule (9)

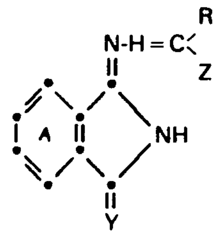

(9)

et on fait réagir le produit de condensation obtenu en présence d'un agent capable de céder du zinc, du cadmium, du manganèse, du cobalt, du fer, du cuivre ou du nickel ou un mélange de ces métaux, avec un composé de formule (10)

$$H - Q \qquad (10)$$

ou

b) on condense un composé de formule (10) avec un ester orthocarboxylique de formule (7) ou une amidine de formule (8) ou un sel d'une amidine de formule (8) avec un acide minéral ou organique, en présence d'un agent capable de céder du zinc, du cadmium, du manganèse, du cobalt, du fer, du cuivre ou du nickel ou un mélange de ces metaux, et on fait réagir le produit de condensation obtenu avec une hydrazone de formule (6); dans les formules (6 à 10) précédentes les symboles A, R, Y et Q ont les significations qui sont été données à propos de la formule (1), $R_2$ représente un radical alkyle, aralkyle ou aryle, $R_2'$ un atome d'hydrogène ou un radical alkyle ou aryle, et Z un radical $-OR_2$ ou un radical $-NHR_2'$ dans lesquels $R_2$ et $R_2'$ ont les significations précédemment données.

2. Procédé selon la revendication 1, caractérisé en ce qu'on utilise, comme composé de formule (10), un composé de ce genre qui répond à l'une des formules (15) et (16)

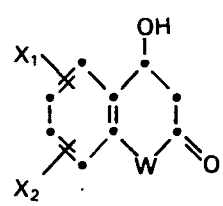

(15)

et

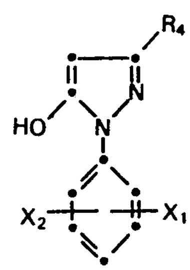

(16),

dans lesquelles W représente O, S, ou NH, $X_1$ représente un atome d'hydrogène, de chlore ou de brome, un radical nitro, trifluorométhyle, carbamoyle ou sulfamoyle, zun radical alkyle, alkoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, $X_2$ représente un atome d'hydrogène, de chlore ou de brome ou un radical alkyle ou alcoxy en $C_1$-$C_4$ et $R_4$ représente un alkyle en $C_1$-$C_4$, un alcoxycarbonyle en $C_2$-$C_6$ ou un carbamoyle.

3. Procédé selon la revendication 1, caractérisé en ce qu'on utilise, comme composé de formule (10), un composé de ce genre qui répond à la formule (18)

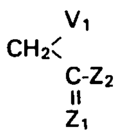 (18)

dans laquelle $V_1$ représente un radical acétyle, benzoyle, cyano ou carbamoyle, un radical alkylcarbamoyle en $C_2$-$C_6$, un radical benzylcarbamoyle ou un radical répondant à la formule (19)

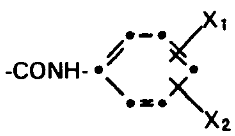 (19)

(dans laquelle $X_1$ représente un atome d'hydrogène, de chlore ou de brome, un radical nitro. trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, et $X_2$ représente un atome d'hydrogène, de chlore ou de brome ou un radical alkyle ou alcoxy en $C_1$-$C_4$), $Z_1$ représente un atome d'oxygène ou de soufre et $Z_2$ représente un radical phényle ou un radical de formule (20)

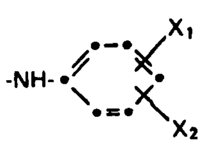 (20)

4. Procédé selon la revendication 1, caractérisé en ce qu'on utilise, comme composé de formule (10), une amine répondant à la formule (21)

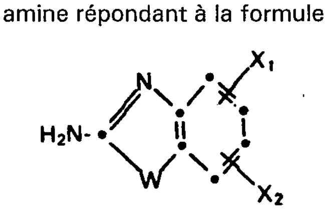 (21)

dans laquelle W représente un atome d'oxygène ou de soufre ou un radical NH, $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, et $X_2$ représente un atome d'hydrogène, de chlore ou de brome, ou un radical alkyle ou alcoxy en $C_1$-$C_4$.

5. Procédé selon la revendication 1, caractérisé en ce qu'on utilise, comme composé de formule (10), un composé thiocarbamoylique répondant à la formule (22)

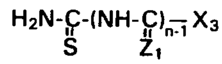 (22)

dans laquelle n et $Z_1$ ont les significations qui ont été données à la revendication 1 et $X_3$ représente un radical amino éventuellement porteur d'un radical hétéroaryle ou d'un radical répondant à la formule suivante:

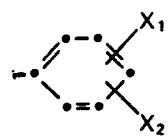

dans laquelle $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, et $X_2$ représente un atome d'hydrogène, de chlore ou de brome, ou un radical alkyle ou alcoxy en $C_1$-$C_4$.

6. Procédé selon la revendication 1, caractérisé en ce qu'on utilise une hydrazone de formule (6) dans laquelle A ne porte pas de substituant supplémentaire et Y représente un radical répondant à l'une des formules (23) et (24)

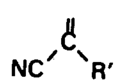 et 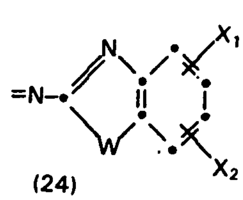

(23) (24)

dans lesquelles $R_1'$ représente un radical alcoxycarbonyle, alkylcarbamoyle, carbamoyle, thiocarbamoyle ou sulfamoyle, un radical benzylcarbamoyle un radical phénylsulfamoyle ou phénylsulfonyle éventuellement porteur d'atomes d'halogènes ou de radicaux alkyles en $C_1$-$C_4$, mais plus particulièrement un radical répondant à l'une des formules (17) et (21a)

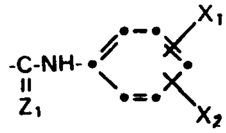 (17)

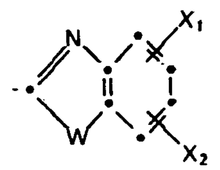

(21a)

dans lesquelles $Z_1$ a la signification qui a été donnée à la revendication 1, $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, $X_2$ représente un atome d'hydrogène, de chlore ou de brome, ou un radical alkyle ou alcoxy en $C_1$-$C_4$, et W représente O, S ou NH.

7. Procédé selon la revendication 1, caractérisé en ce qu'on utilise un ester orthocarboxylique de formule (7) ou une amidine de formule (8) dans lesquels R représente un atome d'hydrogène ou un radical méthyle, $R_2$ représente un radical alkyle en $C_1$-$C_4$, et $R_2'$ représente un atome d'hydrogène, un alkyle en $C_1$-$C_4$ ou un phényle.

8. Procédé selon la revendication 1, caractérisé en ce qu'on utilise des sels de nickel.

9. Procédé selon la revendication 1, caractérisé en ce qu'on utilise, comme solvant polaire, le diméthylformamide ou la N-méthylpyrrolidone.

10. Procédé selon la revendication 1, caractérisé en ce qu'on effectue la réaction à des températures comprises entre 100 et 200°C.

11. Dérivés de l'iso-indoline qui répondent à la formule (9)

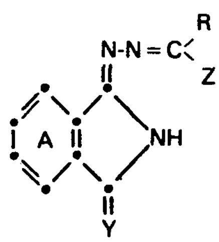

(9)

dans laquelle:
le noyau A peut porter des substituants supplémentaires,

Y représente le radical d'un composé à méthylène actif ou d'une amine aromatique isocyclique ou hétérocyclique,

R représente un atome d'hydrogène ou un radical alkyle ou aryle,

Z représente un radical $-OR_2$ ou $-NHR_2'$,

$R_2$ représente un radical alkyle, aralkyle ou aryle et

$R_2'$ représente un atome d'hydrogène ou un radical alkyle ou aryle.

12. Dérivés de l'iso-indoline selon la revendication 11 dans lesquels A ne porte pas de substituant supplémentaire, R représente un atome d'hydrogène ou un radical méthyle, Z représente un radical alcoxy en $C_1$-$C_4$ et Y représente un radical répondant à l'une des formules (23) et (24)

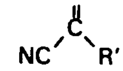

et

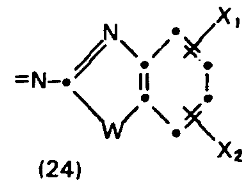

(23) (24)

dans lesquelles R' représente un radical alcoxycarbonyle, alkylcarbamoyle, carbamoyle, thiocarbamoyle ou sulfamoyle, un radical benzylcarbamoyle, un radical phénylsulfamoyle ou phénylsulfonyle éventuellement porteur d'atomes d'halogènes ou de radicaux alkyles en $C_1$-$C_4$, mais plus particulièrement un radical répondant à l'une des formules (17) et (21a)

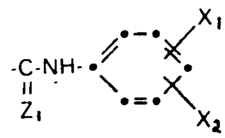

(17)

et

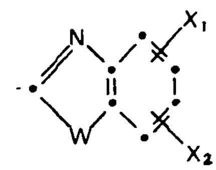

(21a)

dans lesquelles $Z_1$ représente un atome d'oxygène ou de soufre, $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, $X_2$ représente un atome d'hydrogène, de chlore ou de brome, ou un radical alkyle ou alcoxy en $C_1$-$C_4$, et W représente O, S ou NH.

13. Complexes métalliques 1:1 dans lesquels le métal est Zn, Cd, Mn, Co, Fe, Cu ou Ni, ou un mélange de ces métaux, et qui dérivent d'azines répondant à la formule (1)

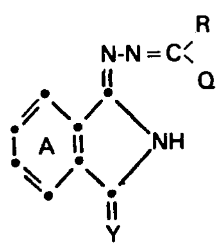

(1)

dans laquelle:
le noyau A peut porter d'autres substituants,

Y représente le radical d'un composé à méthylène actif ou d'une amine aromatique isocyclique ou hétérocyclique,

R représente un atome d'hydrogène ou un radical alkyle ou aryle et

Q représente un radical répondant à l'une des formules (3) et (4)

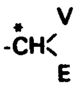
(3)

et

-NH-T
(4)

dans lesquelles

E représente un hétérocycle pentagonal ou hexagonal contenant un atome d'azote en position $\beta$ par rapport à l'atome C* et éventuellement condensé, un radical acyle ou un radical carbamoyle ou thiocarbamoyle éventuellement substitué,

V représente un radical acyle, cyano ou nitro ou un radical carbamoyle éventuellement substitué et

T représente un radical hétérocyclique pentagonal ou hexagonal, contenant un atome d'azote en position $\beta$ par rapport au groupe NH et éventuellement condensé, ou un radical répondant à la formule (5)

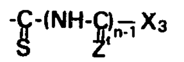
(5)

dans laquelle

$Z_1$ représente un atome d'oxygène ou de soufre,

n désigne le nombre 1 ou le nombre 2 et

$X_3$ représente un radical alkyle, cyclo-alkyle, aralkyle, aryle ou hétérocyclique, ou un radical amino éventuellement porteur d'un radical alkyle, cycloalkyle, aralkyle, aryle ou hétéroaryle.

14. Complexes métalliques 1:1 dérivant d'azines de formule (1) selon la revendication 13, dans lesquels A ne porte pas de substituant supplémentaire et Q représente un radical répondant à la formule (18)

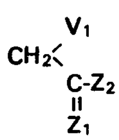
(18)

dans laquelle $V_1$ représente un radical acétyle, benzoyle, cyano ou carbamoyle, un radical alkylcarbamoyle en $C_2$-$C_6$, un radical benzyl-carbamoyle ou un radical répondant à la formule (19)

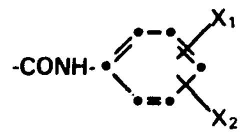
(19)

dans laquelle $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$-$C_4$, un radical alcanoylamino, alkylcarbamoyle ou alcoxycarbonyle en $C_2$-$C_6$, ou un radical phénoxy, benzoylamino, phénylcarbamoyle, phénylsulfamoyle ou phénylazo éventuellement porteur d'atomes de chlore ou de brome ou de radicaux méthyles, et $X_2$ représente un atome d'hydrogène, de chlore ou de brome ou un radical alkyle ou alcoxy en $C_1$-$C_4$, $Z_1$ représente un atome d'oxygène ou de soufre et $Z_2$ un radical phényle ou un radical répondant à la formule (20)

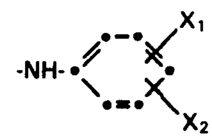
(20)

dans laquelle $X_1$ et $X_2$ ont les significations qui viennent d'être données.

15. Complexes métalliques 1:1 dérivant d'azines de formule (1) selon la revendication 13, dans lesquels A ne porte pas de substituant supplémentaire et Q représente un radical répondant à la formule:

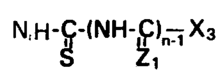

dans laquelle n, $X_3$ et $Z_1$ ont les significations données à la revendication 13.

16. Procédé de pigmentation de matières organiques à haut poids moléculaire, procédé caractérisé en ce qu'on utilise les complexes métalliques selon la revendication 13.

17. Matières organiques à haut poids moléculaire qui contiennent un complexe métallique selon la revendication 13.